# EUROPEAN PATENT SPECIFICATION

(11) **EP 3 693 476 B1**
(45) Date of publication and mention of the grant of the patent: **18.10.2023**
(21) Application number: 20154250.3
(22) Date of filing: 22.02.2013
(51) Int. Cl.: C12Q 1/6886

(54) **CANCER PATIENT SELECTION FOR ADMINISTRATION OF WNT SIGNALING INHIBITORS USING RNF43 MUTATION STATUS**
KREBSPATIENTENAUSWAHL ZUR VERABREICHUNG VON WNT-SIGNALWEG-INHIBITOREN UNTER VERWENDUNG EINER RNF43-MUTATIONSTATUS
SÉLECTION DE PATIENTS ATTEINTS D'UN CANCER POUR L'ADMINISTRATION D'INHIBITEURS DE LA VOIE DE SIGNALISATION DE WNT AU MOYEN DE L'ÉTAT MUTATIONNEL DE RNF43

(30) Priority: 28.02.2012 US 201261604290 P
(43) Date of publication of application: 12.08.2020
(62) Divisional of application: 13707527.1
(73) Proprietor: Novartis AG, 4056 Basel (CH)
(72) Inventor: CONG, Feng, Cambridge, MA 02139 (US); HAO, Huaixiang, Cambridge, MA 02139 (US); HSIEH, Hsin-i, San Diego, CA 92121 (US); LIU, Jun, San Diego, CA 92121 (US); NG, Nicholas, San Diego, CA 92121 (US); JIANG, Xiaomo, Cambridge, MA 02139 (US)
(74) Representative: Mueller, Philippe

(56) References cited:
- WO-A1-2011/004379
- WO-A2-2004/032838
- WO-A2-2006/017318
- J. LIU ET AL: "PD08-11: Targeting Porcupine, a Critical Node for Wnt Signalling in Cancer.", CANCER RESEARCH, vol. 71, no. 24 Supplement, 15 December 2011 (2011-12-15), pages PD08-11, XP055061279, ISSN: 0008-5472, DOI: 10.1158/0008-5472.SABCS11-PD08-11
- BON-KYOUNG KOO ET AL: "Tumour suppressor RNF43 is a stem-cell E3 ligase that induces endocytosis of Wnt receptors", NATURE, vol. 488, no. 7413, 15 August 2012 (2012-08-15), pages 665-669, XP055061276, ISSN: 0028-0836, DOI: 10.1038/nature11308
- Guo Dong ET AL: "Expression of wnt signaling target genes in colorectal cancer progression", AACR annual meeting, 1 January 2007 (2007-01-01), pages 1-3, XP055783273, Retrieved from the Internet: URL:https://cancerres.aacrjournals.org/con tent/67/9_Supplement/1270 [retrieved on 2021-03-09]

## Description

### FIELD OF THE INVENTION

The invention relates generally to identifying cancer patients who are predicted to benefit from Wnt inhibitor therapy. The invention also relates to a Wnt inhibitor or a pharmaceutical composition comprising a Wnt inhibitor for use in a specifically selected patient.

### BACKGROUND OF THE INVENTION

In biology and medicine, the canonical Wnt/β-catenin pathway is a series of intracellular signaling events involving secreted Wnt ligands, cell surface receptors and transcription co-activator β-catenin, as well as many other effectors and regulators of these core protein components. In the absence of Wnt ligands, β-catenin is constantly phosphorylated by a multi-protein complex that triggers its poly-ubiquitination and proteasomal degradation. Upon the binding of Wnt protein to its receptors, cytosolic β-catenin is stabilized through inhibition of the destruction complex and translocates to the nucleus to activate transcription of Wnt target genes.

The primary receptors for Wnts are the Frizzled (FZD) family of proteins, with LRP5 or LRP6 (LDL receptor related proteins 5 and 6) as essential co-receptors for Wnt/β-catenin signaling. Nusse R (2005), Cell Research 15(1):28-32. Frizzled receptors are seven-span transmembrane molecules with a long cysteine-rich domain. LRP5/6 co-receptors are single-pass transmembrane proteins. Huang H-C and Klein PS (2004) Genome Biol. 5(7):234. LRP6 is dominant as compared with LRP5, which is only required for adult bone homeostasis. McDonald BT et al. (2009) Developmental Cell 17(1):9-26.

The non-canonical Wnt signaling is β-catenin independent. Frizzled receptors participate in the non-canonical Wnt signaling, but LRP6 co-receptor is not essential for non-canonical pathway activity. There are at least two non-canonical Wnt signaling pathways, the planar cell polarity (PCP) pathway and the Wnt calcium releasing pathway.

Wnt/β-catenin signaling is important for regulating cell growth and differentiation during embryonic development. In adults, Wnt signaling promotes tissue homeostasis and its dysregulation has been implicated in a variety of human diseases, especially cancer. Nusse R (2005) Cell Research 15(1):28-32.

Aberrant over-activation of Wnt pathway is often important for tumorigenesis of colorectal carcinomas. Other cancer types have also been shown to be associated with abnormal Wnt signaling. These other cancer types include pancreatic cancer, liver cancer, breast cancer and skin cancer. An elevated activity in the Wnt/PCP non-canonical pathway has also been implicated in tumorigenesis.

Wnt signaling antagonists have been developed for treating Wnt-dependent tumors. WO2006/017318 discloses methods for treating cancer using anti-Wnt16 antibody that inhibit WNT16 signaling. WO2011/004379 discloses a method of killing cancer cells expressing NCAM and optionally FZD7 comprising contacting the cancer cell with a cytotoxic moiety and an NCAM targeting moiety, thereby killing the cancer cell. WO2004/032838 discloses a method of inhibiting the growth of cancer cells that overexpress a Wnt protein, the method of WO2004/032838 comprises contacting the cell with an agent that inhibits binding of the Wnt protein to a Frizzled receptor, wherein the agent is anti-Wnt1 or anti-Wnt2 or anti-Frizzled antibody. Guo et al (AACR annual meeting, 1 January 2007, pages 1-3) disclose RNF43 as a putative Wnt target gene, implying that RNF43 is downstream in the Wnt signalling pathway. Many Wnt inhibitors, such as Porcupine inhibitors, tankyrase inhibitors, Frizzled antibodies and LRP6 antibodies, are being developed for cancer treatment. However, most of these Wnt inhibitors target upstream protein components of the Wnt signaling pathway. These Wnt inhibitors would not inhibit Wnt signaling in tumors with mutations in genes that are downstream in the Wnt pathway and so are often not effective against tumors with oncogenic mutations in the downstream Wnt pathway genes such as APC (adenomatous polyposis coli), AXIN1/2, and β-catenin.

This lack of tumors identified as having mutations in genes that are upstream in the Wnt pathway has hampered the clinical development of Wnt inhibitors. Thus, there is a need in the art for a method to identify a cancer-associated Wnt pathway mutation in a gene or gene product that is an upstream component of the Wnt pathway.

### SUMMARY OF THE INVENTION

In one aspect, the invention provides a method for selecting a cancer patient for treatment with a Wnt inhibitor, comprising testing a biological sample from the patient for the presence of an inactivating mutation in the RNF43 gene.

In one aspect, the invention provides a Wnt inhibitor or a pharmaceutical composition comprising a Wnt inhibitor for use in the treatment of cancer in a patient, wherein Wnt inhibitor is administered to the patient on the basis that an inactivating mutation in the RNF43 gene is detected in a biological sample obtained from the patient, and wherein the presence of an inactivating mutation in the RNF43 gene is detected using a method selected from a list consisting of DNA sequencing methods, sequencing of genomic DNA, cDNA or RNA from a cancer tissue, by hybridization, polymerase chain reaction, polyacrylamide gel analysis, chromatography or spectroscopy, and screening for an altered protein product encoded by the gene, e.g. via immunoblot, Western blot, enzyme-linked immunosorbant assay (ELISA), radioimmunoassay (RIA), immunoprecipitation, immunohistochemistry, immunofluorescence, fluorescence activated cell sorting (FACS) and immunofluorescence microscopy.

These aspects of the invention and further embodiments are defined in the attached claims.

### BRIEF DESCRIPTION OF THE DRAWINGS

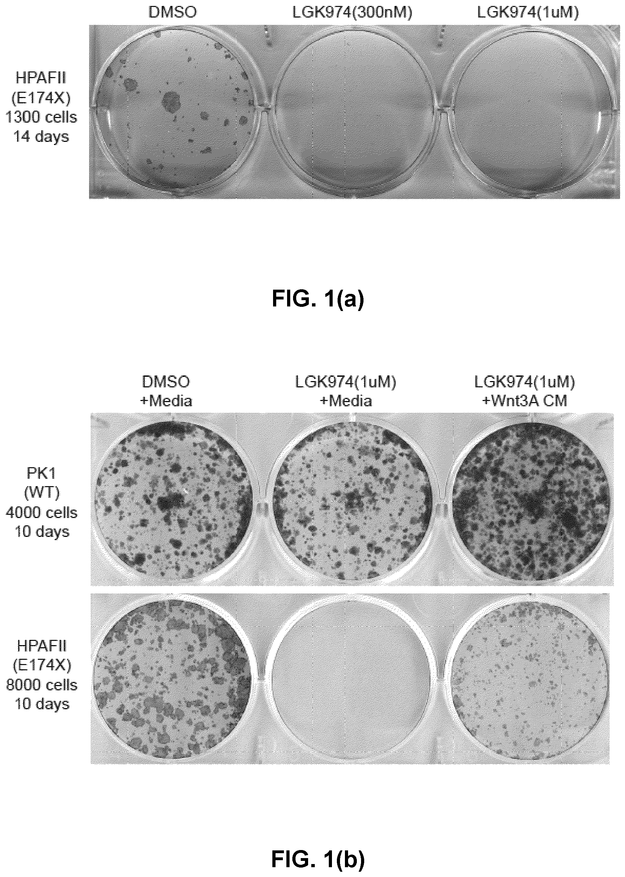

FIG. 1 is a photograph showing colony formation assays with LGK974 treatment. FIG. 1(a) shows a HPAFII cell colony formation assay in the presence of LGK974 at two concentrations (300 nM and 1 µM). 1300 cells were seeded into media with indicated treatment and the media were replaced every 5 days until crystal violet staining at day 14. LGK974 inhibits colony formation of HPAFII cells bearing non-sense mutation at RNF43 gene. FIG. 1(b) shows PK1 (having a functional RNF43 protein) and HPAFII (having a nonfunctional RNF43 protein) cell colony formation assays in the presence of 1 µM LGK974 alone or together with daily addition of 10% Wnt3a conditioned media (CM). RNF43 wild-type PK1 cells showed no sensitivity to LGK974 during the 10 day assay. RNF43 mutant HPAFII cells were inhibited by LGK974 and the inhibition was partially rescued by addition of exogenous Wnt3a, so the growth inhibition by LGK974 was Wnt signaling dependent.

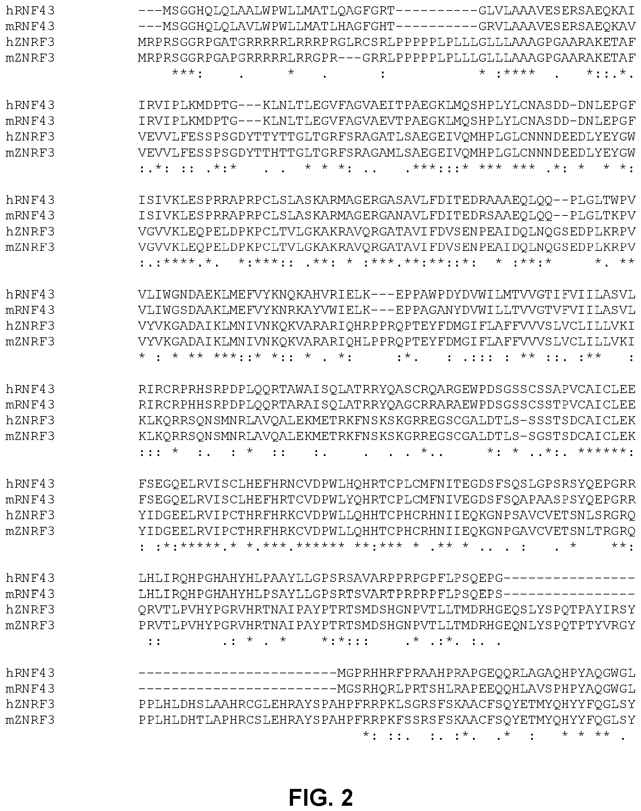

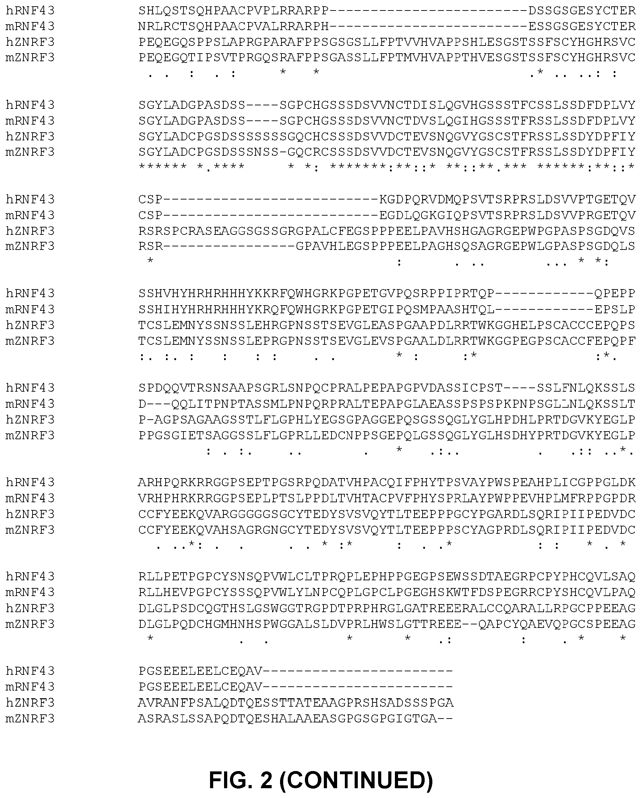

FIG. 2 is a chart showing the alignment of RNF43 protein and ZNRF3 protein to show conserved residues. This information can be used to predict missense mutation that are inactivating mutations. The sequence of human RNF43 protein is also provided in SEQ ID NO:3 and the sequence of human ZNRF3 is also provided in SEQ ID NO:4.

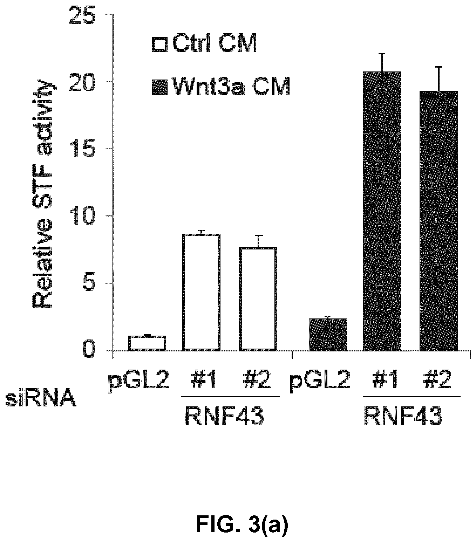

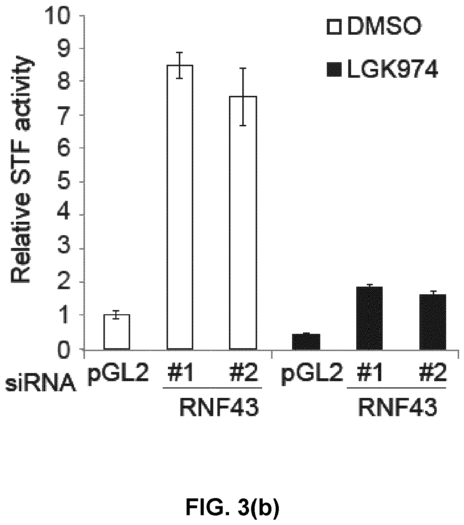

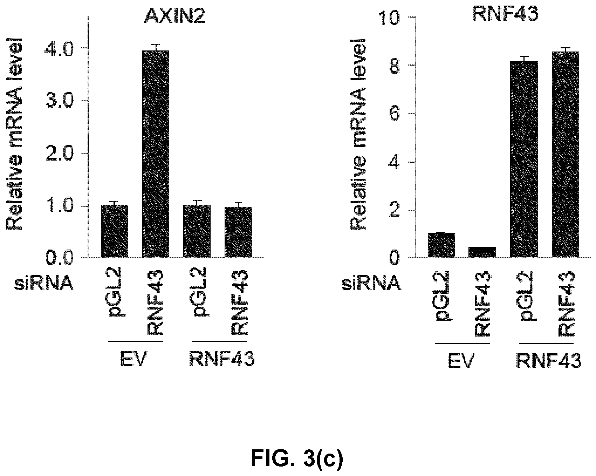

FIG. 3 is a set of bar graphs showing the negative regulation of Wnt signaling by RNF43 in pancreatic cancer cells YAPC, which express wild-type RNF43. FIG. 3(a) shows the depletion of RNF43 increases Super TOPFlash (STF) activity in YAPC pancreatic cancer cell line. YAPC-STF cells were transfected with indicated siRNA in the absence or presence of Wnt3a conditioned medium (CM), and STF luciferase reporter activity was measured. pGL2 siRNA acts as a negative control. FIG. 3(b) shows that RNF43 siRNA-induced activation of STF activity is dependent on endogenous Wnt. YAPC-STF cells were transfected with indicated siRNA, and then treated with DMSO or Porcupine inhibitor LGK974. STF reporter activity was then measured. FIG. 3(c) shows the depletion of RNF43 increases the expression of AXIN2, a β-catenin target gene. YAPC cells expressing empty vector (EV) or siRNA-resistant RNF43 were transfected with indicated siRNA, and Relative mRNA levels of AXIN2 and RNF43 by quantitative RT-PCR.

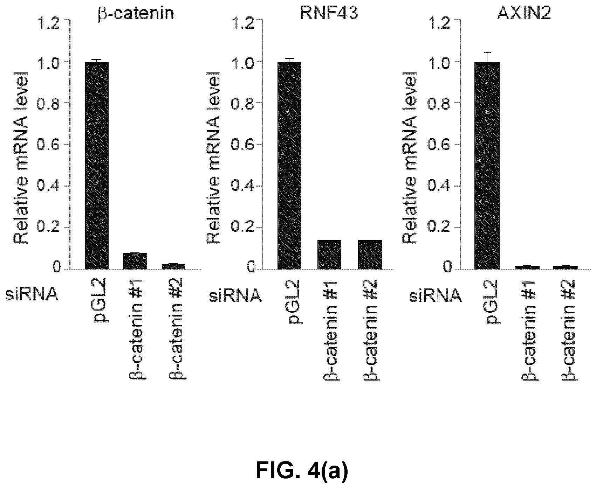

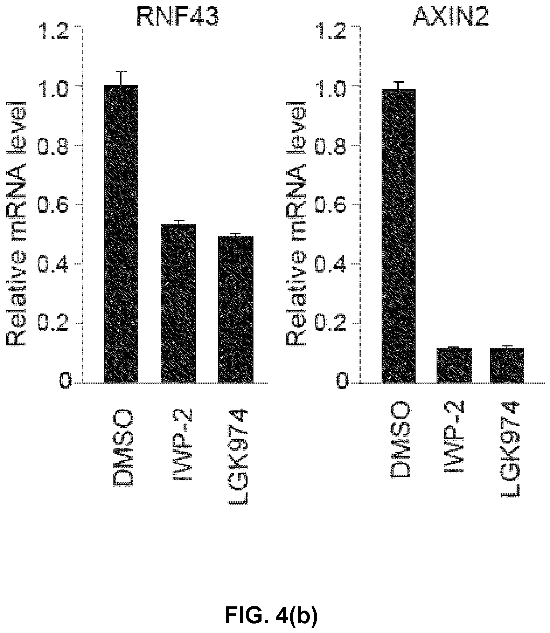

FIG. 4 is a set of bar graphs showing alterations in the mRNA level for several Wnt/β-catenin signaling-related genes. FIG. 4(a) shows that the depletion of β-catenin decreases mRNA level of AXIN2 and RNF43. Cells were treated with siRNA, and relative mRNA levels of indicated genes were analyzed by quantitative RT-PCR. FIG. 4(b) shows that Porcupine inhibitors decreased the expression of RNF43 mRNA and AXIN2 mRNA. YAPC cells were treated with 3µM of IWP2 or 1µM of LGK974, and subjected to gene expression analysis by quantitative RT-PCR.

### DETAILED DESCRIPTION OF THE INVENTION

### Introduction

The technical information set out below may in some respects go beyond the scope of the invention, which is defined by the attached claims. The additional technical information is provided to place the actual invention in a broader technical context and to illustrate possible related technical developments.

The inventors had discovered the function of two negative regulators of Wnt signaling, Zinc/RING finger protein 3 (ZNRF3, Swiss-Prot Q9ULT6, SEQ ID NO:4) and Ring finger protein 43 (RNF43, Swiss-Prot Q68DV7, SEQ ID NO:3). Hao HX et al. (2012) Nature 485(7397): 195-200. RNF43, a cancer-associated RING finger protein, is an ubiquitin ligase that interacts with a nuclear protein, HAP95. Sugiura T et al. (2008) Exp. Cell Res. 314(7):1519-28. ZNRF3 and RNF43 are both homologous cell surface transmembrane E3 ubiquitin ligases for Wnt receptor Frizzled. Both proteins inhibit the cell surface levels of Wnt receptor complex composing Frizzled and LRP6.

The inventors had also shown that ZNRF3 is transcriptionally induced by Wnt pathway activation. Overexpression of ZNRF3 decreased Wnt signaling, while ZNRF3 siRNA or dominant negative ZNRF3 potently increased Wnt signaling. LRP6 phosphorylation was increased upon ZNRF3 inhibition, showing that ZNRF3 acts in the upstream of Wnt pathway. Therefore, ZNRF3 regulates the cellular responsiveness to Wnt ligand stimulation. The inventors confirmed this observation in cellular systems by *in vivo* experiments using both zebrafish and knockout mice. The inventors have also shown that RNF43 is a functional homolog of ZNRF3 and regulates Wnt signaling.

Described herein is a diagnostic use for two homologous transmembrane E3 ubiquitin ligases (RNF43 and ZNRF3), both of which negatively regulate Wnt receptor complex Frizzled/LRP6 levels through Frizzled ubiquitination. Described herein is also the use of inactivating mutations in the RNF43 gene or the ZNRF3 gene as biomarkers for the selection of tumors cells that are susceptible having their growth slowed by inhibitors of the Wnt pathway. In one aspect, the invention provides for the selection of cancer patients for treatment with Wnt inhibitors, where the selection is by the use of inactivating mutations in the RNF43 gene as biomarkers for tumor susceptibility to Wnt inhibitor treatment. It is disclosed herein that RNF43 inactivating mutations such as missense and frame shift mutations are present in primary tumors and cell lines of pancreatic ductal adenocarcinomas (PDAC). Since endogenous wild-type RNF43 regulates Wnt signaling in PDAC cells and inhibition of endogenous RNF43 in PDAC increases Frizzled level and Wnt signaling, the invention provides the identification of an upstream Wnt pathway component (RNF43) as being mutated in cancer.

It is presented herein that cancer cells with RNF43 gene mutations are more sensitive to inhibition of the Wnt pathway. Inhibition of RNF43 in cancer cells leads to increased cell surface levels of Frizzled proteins. Accordingly, enhanced Wnt signaling and cancer cells with mutant RNF43, particularly pancreatic cancer cells, are more sensitive to Wnt antagonists. Thus, the invention provides that RNF43 mutation status can be used as a cancer patient selection strategy for therapeutic administration of Wnt signaling inhibitors.

Wnt inhibitors are useful in pharmaceutical compositions for human or veterinary use where inhibition of the Wnt pathway is indicated, *e.g.,* in the treatment of tumors or abnormal cell growth. The invention provides a Wnt inhibitor or a pharmaceutical composition comprising a Wnt inhibitor for use in a method of treating a cancer patient with a Wnt inhibitor, comprising administering a Wnt inhibitor or a pharmaceutical composition comprising a Wnt inhibitor to a patient on the basis that an inactivating mutation in the RNF43 gene is detected in a biological sample obtained from the patient, wherein the presence of an inactivating mutation in the RNF43 gene is detected using a method selected from a list consisting of DNA sequencing methods, sequencing of genomic DNA, cDNA or RNA from a cancer tissue, by hybridization, polymerase chain reaction, polyacrylamide gel analysis, chromatography or spectroscopy, and screening for an altered protein product encoded by the gene, e.g. via immunoblot, Western blot, enzyme-linked immunosorbant assay (ELISA), radioimmunoassay (RIA), immunoprecipitation, immunohistochemistry, immunofluorescence, fluorescence activated cell sorting (FACS) and immunofluorescence microscopy.

ZNRF3 and RNF43 are functional homologs and ZNRF3 is also mutated in certain type of tumors. The mutational status of ZNRF3 is thus also informative for cancer patient selection for therapeutic administration of Wnt antagonists.

Described herein is also an assay or kit for testing RNF43 gene or ZNRF3 gene mutation status. The assay or kit may be a companion diagnostic assay for use after drug approval of a related Wnt inhibitor.

### RNF43 and Pancreatic tumors

Pancreatic ductal adenocarcinoma (PDAC) is the most common form of pancreatic cancer. PDAC is extremely aggressive and associated with dismal prognosis. Matthaei H et al. (2011) Ann. Surg. Oncol. 18(12):3493-9; Luebke AM et al. (2012) Pancreatology 12(1):16-22; Hezel AF (2006) Genes Dev. 20(10). Pancreatic intraepithelial endoplasia (PanIN), intraductal papillary mucinous neoplasm (IPMN) and mucinous cystic neoplasm (MCN) are considered as precursors for PDAC. Despite of its ductal characteristics, PDAC does not necessarily arise from the ductal compartment.

The Wnt/β-catenin signaling pathway dynamically regulated during pancreatic development and is required for the development of exocrine pancreas. Wells JM (2007) BMC Dev. Biol. 7:4. Inhibition of Wnt/β-catenin signaling disrupts acinar but not islet development of the pancreas. Inducible expression of stabilized β-catenin in the pancreas of adult mice increases the proliferation of mature exocrine cells with minimal effects on the islet cells. Heiser PW et al. (2006) Development 133(10):2023-32. There is growing evidence that Wnt pathway activation may contribute to the maintenance and/or progression of PDAC. Morris JP et al. (2010) Nat. Rev. Cancer 10(10):683-95. Nuclear or cytoplasmic accumulation of β-catenin is observed in a subset of PDAC (Pasca di Magliano M et al. (2007) PLoS One 2(11):e1155), indicating that the pathway is activated. The level of cytosolic and nuclear β-catenin is positively correlated with PanIN grade and development of PDAC (Wang L et al. (2009) Cancer Sci. 101(3):700-6), so β-catenin signaling promotes the progression of PDAC. Depletion of β-catenin decreased proliferation of PDAC cells, showing the importance of β-catenin in the maintenance of transforming phenotype.

RNF43 is frequently mutated in IMPN and MCN (Furukawa T et al.(2011) Sci. Rep. 1:161; Wu J et al. (2011) Proc. Natl. Acad. Sci. USA 108(52):21188-93). IPMN and MCN are precursors to invasive pancreatic ductal adenocarcinoma (PDAC).

The sequence of human RNF43 is known in the art. Full length human RNF43 cDNA (NM_017763.4) can purchased from commercial sources (*e.g.,* Open Biosystems, Glastonbury, CT, USA 06033). For further information on RNF43, see U.S. Pat. No. 7,425,612. The TAT179 polypeptide is identical to the RNF43 extracellular region after the signal peptide. See, international patent application WO2003/024392, granted in Europe as EP1487877B1.

Recently, RNF43 was proposed to be a tumor suppressor in cystic pancreatic tumors. Wu J et. al. (2011) Proc. Natl. Acad. Sci. 108:2188. In a whole exome sequencing study, 6 of 8 intraductal papillary mucinous neoplasms (IPM Ns) and 3 of 8 mucinous cystic neoplasms (MCNs) were shown to harbor inactivating mutations of RNF43. The preponderance of inactivating mutations in RNF43 and loss of heterozygosity (LOH) of its locus establish RNF43 as a tumor suppressor in both IPMNs and MCNs. In their report, Wu *et al.* provided no functional study of RNF43.

More recently, intestinal-specific deletion of both Znrf3 and Rnf43 were shown to induce overproliferation of intestinal crypts and formation of intestinal adenoma in mice. Koo BK et al. (2012) Nature 488(7413):665-9. Also, mutations of RNF43 have been identified in different tumors, including cystic pancreatic tumors. These studies show that RNF43 acts as a negative regulator of Wnt/β-catenin signaling like ZNRF3.

However, a cellular system in which RNF43 is important has not been identified, and *in vitro* loss of function study of RNF43 has not been possible. Thus, the physiological relevance of RNF43 mutation in cancer has previously been unknown.

### The Wnt/β-catenin pathway

The evolutionarily conserved Wnt/β-catenin signaling pathway is important for embryonic development and adult tissue homeostasis. Logan CY and Nusse R (2004) Annu. Rev. Cell. Dev. Biol. 20:781-810; Clevers H (2006) Cell 127(3):469-80. Wnt signaling regulates the turn-over of the transcription cofactor β-catenin and controls key developmental gene expression programs. MacDonald BT et al. (2009) Dev. Cell. 17(1):9-26. In the absence of Wnt pathway activation, cytosolic β-catenin is associated with β-catenin destruction complex, which contains multiple proteins including adeomatous polyposis coli (APC), AXIN, and glycogen synthase kinase 3α/β (GSK3α/β). In this complex, β-catenin is constitutively phosphorylated by GSK3, and phosphorylated β-catenin is degraded by the ubiquitin proteasome pathway. Wnt signal is received by its two receptors, Frizzled and LRP5/6, which leads to dissociation of β-catenin destruction complex. Stabilized β-catenin enters the nucleus, binds to TCF family of transcription factors, and turns on transcription.

Aberrant activation of Wnt/β-catenin signaling has been implicated in tumorigenesis, and many downstream components of Wnt pathway are mutated in cancers. Truncation mutations of APC are found in 80% of colorectal cancer. Stabilization mutations of β-catenin and loss of function mutations of AXIN1/2 are also found in various cancers. Despite intense research, targeting β-catenin signaling in cancers harboring downstream pathway mutation remains challenging due to lack of tractable targets. On the other hand, there are several tractable targets in the upstream of Wnt signaling pathway. Various agents targeting upstream Wnt signaling are being developed, including LRP6 antibody (Ettenberg S et al. (2010) Proc. Natl. Acad. Sci. USA 107(35):15473-8), Frizzled antibody (Gurney A et al. (2012) Proc. Natl. Acad. Sci. USA 109(29): 11717-22), and Porcupine inhibitor (Chen B et al. (2009) Nat. Chem. Biol. 5(2): 100-7). However, clinical development of these agents has previously been difficult, since these molecules would not inhibit β-catenin signaling in tumors with downstream mutation and it has been challenging to identify human tumors addicted to ligand-induced Wnt/β-catenin signaling.

### Definitions

"Cancer", as described in U.S. Pat. No. 8,093,011 and U.S. Pat. No. 8,093,011 is a generic name for a wide range of cellular malignancies characterized by unregulated growth, lack of differentiation, and the ability to invade local tissues and metastasize. These neoplastic malignancies affect, with various degrees of prevalence, every tissue and organ in the body. Thus, the term "cancer" as used herein refers to the presence of cells possessing characteristics typical of cancer-causing cells, such as uncontrolled proliferation, immortality, metastatic potential, rapid growth and proliferation rate, and certain characteristic morphological features. Often, cancer cells will be in the form of a tumor, but such cells may exist alone or may circulate in the blood stream as independent cells, such as leukemic cells.

"Abnormal cell growth" refers to cell growth that is independent of normal regulatory mechanisms (*e.g.,* loss of contact inhibition). This includes the abnormal growth of: (1) tumor cells (tumors) that proliferate by overactivity of a Wnt pathway in the cell; (2) benign and malignant cells of other proliferative diseases in which aberrant overactivity of a Wnt pathway occurs in the cell; (4) any tumors that proliferate by overactivity of a Wnt pathway in the cell; (5) any tumors that proliferate by overactivity of a Wnt pathway in the cell; and (6) benign and malignant cells of other proliferative diseases in which aberrant overactivity of a Wnt pathway occurs.

A "biomarker" is anything that can be used as an indicator of a particular disease state or some other physiological state of an organism, such as a human. A biomarker can be the presence of a gene, an allele of a gene, a measure of gene expression, a protein, or functional effect of the protein activity that can be measured and correlated with a physiological state. Biomarkers are used in medicine as laboratory parameters that a physician can use to help make decisions in making a diagnosis and selecting a course of treatment.

A "loss of heterozygosity" (LOH) is a deletion of genetic material (DNA) which almost all varieties of cancer undergo, as compared to normal cells. See, U.S. Pat. No. 7,718,364. The loss of genetic material from cancer cells can result in the selective loss of one of two or more alleles of a gene at a particular locus on the chromosome, where the gene may affect cell growth. Due to the genetic heterogeneity or DNA polymorphism, many of the paired alleles of genes differ from one another. When the two alleles are identical, the individual is said to be homozygous for that pair of alleles at that particular locus. Alternatively, when the two alleles are different, the individual is heterozygous at that locus. Typically, both alleles are transcribed and ultimately translated into either identical proteins in the homozygous case or different proteins in the heterozygous case. If one of a pair of heterozygous alleles is lost due to deletion of DNA from one of the paired chromosomes, only the remaining allele will be expressed and the affected cells will be functionally homozygous. This situation is termed as "loss of heterozygosity" (LOH) or reduction to homozygosity. Through the use of DNA probes, DNA from an individual's normal cells can be compared with DNA extracted from the same individual's tumor cells and LOH can be identified using experimental techniques well known in the art. Alternatively, LOH can be assayed by demonstrating two polymorphic forms of a protein in normal heterozygous cells, and only one form in cancer cells where the deletion of an allele has occurred. See, for example, Lasko et al. (1991) Annu. Rev. Genet. 25:281-314. Frequent LOH on specific chromosomal regions has been reported in many kinds of malignancies, which shows the existence of putative tumor suppresser genes or tumor-related genes on or near these loci. Thus, LOH analysis is a powerful tool to search for a tumor suppresser gene by narrowing and identifying the region where a putative gene exists. See, Vogelstein et al. (1988) New Engl. J. Med. 319(9):525-532; Fearon et al. (1990) Cell 61:759-767; and Friend et al., Nature 323:643-646 (1986). Analyses have identified many kinds of candidate tumor suppressor or tumor-related genes. See, Call et al. (1990) Cell 60:509-520; Kinzler et al. (1991) Science 253:661-665; and Baker et al. (1989) Science 244:217-221.

A "loss of function" (LOF) mutation is a mutation or allele of a gene, the result of which is that the gene product (such as the encoded protein) has less than normal or no function in a cell or organism (including a human cell or human being). When the allele has a complete loss of function (null allele) it is often called an amorphic mutation. Phenotypes associated with loss of function mutations are often recessive.

A "substitution" is a mutation that exchanges one base for another (i.e., a change in a single "chemical letter" such as switching an A to a G). Such a substitution could (1) change a codon to one that encodes a different amino acid and cause a small change in the protein produced (for example, sickle cell anemia is caused by a substitution in the beta-hemoglobin gene, which alters a single amino acid in the protein produced; (2) change a codon to one that encodes the same amino acid and causes no change in the protein produced ("silent mutations"); or (3) change an amino-acid-coding codon to a single "stop" codon and cause an incomplete protein (an incomplete protein is usually nonfunctional).

An "insertion" is a mutation in which extra base pairs are inserted into a place in the DNA.

A "deletion" is a mutation in which a section of DNA is lost, or deleted.

A "frameshift" is a mutation caused by insertions or deletions) of a number of nucleotides that is not evenly divisible by three from a DNA sequence. Due to the triplet nature of gene expression by codons, the insertion or deletion can change the reading frame (the grouping of the codons), resulting in a completely different translation from the original. This often generates truncated proteins that result in loss of function.

"Sanger sequencing" (named after its inventor Frederick Sanger) is the chain-terminator method of sequencing polynucleotides. A feature of the Sanger sequencing method is the use of dideoxynucleotide triphosphates (ddNTPs) as DNA chain terminators. The Sanger sequencing method is often an automated method.

"Next generation sequencing" methods are a group recently developed high-throughput sequencing methods that parallelize the sequencing process, producing thousands or millions of sequences at once. The combination of the increase in data generated, coupled with lowered costs required to generate these data, has made this technology be recognized by those of skill in the art as a tractable, general purpose tool.

The term "treating" as used herein, unless otherwise indicated, means reversing, alleviating, inhibiting the progress of, or preventing, either partially or completely, the growth of tumors, tumor metastases, or other cancer-causing or neoplastic cells in a patient. The term "treatment" as used herein, unless otherwise indicated, refers to the act of treating.

The phrase "a method of treating" or its equivalent, when applied to cancer treatment, refers to a procedure or course of action that is designed to reduce or eliminate the number of cancer cells or to alleviate the symptoms of a cancer. "A method of treating" cancer or another proliferative disorder does not necessarily mean that the cancer cells or other disordered cells will actually be eliminated, that the number of cells or disorder will actually be reduced, or that the symptoms of a cancer or other disorder will actually be alleviated. Often, a method of treating cancer will be performed even with a low likelihood of success, but which, given the medical history and estimated survival expectancy, is nevertheless deemed an overall beneficial course of action.

A "therapeutically effective agent" is a composition that will elicit the biological or medical response of a tissue, system, animal or human that is being sought by the researcher, veterinarian, medical doctor or other clinician.

A "therapeutically effective amount" or "effective amount" is the amount of the subject compound or combination that will elicit the biological or medical response of a tissue, system, animal or human that is being sought by the researcher, veterinarian, medical doctor or other clinician.

An "inhibitor" is a compound that inhibits (*e.g.,* antagonizes, reduces, decreases, blocks, reverses, or alters) the effect of a naturally occurring or reference compound as described above. Such inhibitors can include any compound, protein, peptide, or nucleic acid (including ribozymes and antisense) or product of drug/compound/peptide design or selection that provides the antagonistic effect.

An "isolated" polynucleotide, or an isolated nucleic acid molecule, is a nucleic acid molecule that has been removed from its natural milieu (*i.e.,* that has been subject to human manipulation), its natural milieu being the genome or chromosome in which the nucleic acid molecule is found in nature. As such, "isolated" does not necessarily reflect the extent to which the nucleic acid molecule has been purified, but shows that the molecule does not include an entire genome or an entire chromosome in which the nucleic acid molecule is found in nature. Polynucleotides such as those used in a method of the invention to detect genes (*e.g.,* by hybridization to a gene) are typically a portion of the target gene that is suitable for use as a hybridization probe or PCR primer for the identification of a full-length gene (or portion thereof) in a given sample (*e.g.,* a cell sample). An isolated nucleic acid molecule can include a gene or a portion of a gene (*e.g.,* the regulatory region or promoter). An isolated nucleic acid molecule that includes a gene is not a fragment of a chromosome that includes such gene, but rather includes the coding region and regulatory regions associated with the gene, but no additional genes naturally found on the same chromosome. An isolated nucleic acid molecule can also include a specified nucleic acid sequence flanked by (*i.e.,* at the 5' end or the 3' end of the sequence, or both) additional nucleic acids that do not normally flank the specified nucleic acid sequence in nature (*i.e.,* heterologous sequences). Isolated nucleic acid molecule can include DNA, RNA (*e.g.,* mRNA), or derivatives of either DNA or RNA (*e.g.,* cDNA). Although the phrase "nucleic acid molecule" primarily refers to the physical nucleic acid molecule and the phrase "nucleic acid sequence" primarily refers to the sequence of nucleotides on the nucleic acid molecule, the two phrases can be used interchangeably, especially with respect to a nucleic acid molecule, or a nucleic acid sequence, being capable of encoding a protein. An isolated nucleic acid molecule can be produced using recombinant DNA technology (*e.g.,* polymerase chain reaction (PCR) amplification, cloning) or chemical synthesis.

A "probe" (oligonucleotide probe) is a nucleic acid molecule which typically ranges in size from about 50-100 nucleotides to several hundred nucleotides to several thousand nucleotides in length. Therefore, a probe can be any suitable length for use in an assay described herein, including any length in the range of 50 to several thousand nucleotides, in whole number increments. Such a molecule is typically used to identify a target nucleic acid sequence in a sample by hybridizing to such target nucleic acid sequence under stringent hybridization conditions. Hybridization conditions are known in the art. See, for example, Sambrook et al.(1989) Molecular Cloning: A Laboratory Manual. Cold Spring Harbor Labs Press.

"Primers" are also nucleic acid sequences. PCR primers are typically oligonucleotides of fairly short length (*e.g.,* 8-30 nucleotides) that are used in polymerase chain reactions. PCR primers and hybridization probes can readily be developed and produced by those of skill in the art, using sequence information from the target sequence. See, Sambrook et al. (1989) Molecular Cloning: A Laboratory Manual. Cold Spring Harbor Labs Press.

The terms "test sample" or "patient sample" can be used generally to refer to a sample of any type which contains cells or products that have been secreted from cells to be evaluated by the method of the invention, including but not limited to, a sample of isolated cells, a tissue sample or a bodily fluid sample. A sample of isolated cells is a specimen of cells, typically in suspension or separated from connective tissue which may have connected the cells within a tissue *in vivo,* which have been collected from an organ, tissue or fluid by any suitable method which results in the collection of a suitable number of cells for evaluation by the method of the invention. The cells in the cell sample are not necessarily of the same type, although purification methods can be used to enrich for the type of cells that are preferably evaluated. Cells can be obtained, for example, by scraping of a tissue, processing of a tissue sample to release individual cells, or isolation from a bodily fluid.

A "tissue sample", although similar to a sample of isolated cells, is defined herein as a section of an organ or tissue of the body which typically includes several cell types, optionally with cytoskeletal structures that hold the cells together. The term "tissue sample" may be used, in some instances, interchangeably with a "cell sample", although term "tissue sample" may more often used to designate a more complex structure than a cell sample. A tissue sample can be obtained by a biopsy, for example, including by cutting, slicing, or a punch.

A "bodily fluid sample", like the tissue sample, contains the cells to be evaluated, and is a fluid obtained by any method suitable for the particular bodily fluid to be sampled. Bodily fluids suitable for sampling include, but are not limited to, blood, mucous, seminal fluid, saliva, sputum, bronchial lavage, breast milk, bile and urine.

A "control level" is a control level of heterozygosity, which can include a level that is correlated with sensitivity to the Wnt inhibitor or a level that is correlated with resistance to the Wnt inhibitor. Therefore, it can be determined, as compared to the control or baseline level of loss of heterozygosity, whether a patient sample is more likely to be sensitive to or resistant to the Wnt inhibitor therapy (*e.g*., a good responder or responder (one who will benefit from the therapy), or a poor responder or non-responder (one who will not benefit or will have little benefit from the therapy). More specifically, a "control level" can denote a control level of heterozygosity; a DNA, cDNA or RNA sequence showing a wild-type, normal or baseline status; a control level of RNF43 mRNA or activity; a control level of ZNRF3 mRNA or activity, the functional effect of RNF43 gene loss or ZNRF3 gene loss, which can include a level, sequence or effect that is correlated with sensitivity to the Wnt inhibitor or a level that is correlated with resistance to the Wnt inhibitor. Therefore, it can be determined, as compared to the control heterozygosity; a DNA, cDNA or RNA sequence showing a wild-type, normal or baseline status; normal or baseline RNF43 mRNA or activity; normal or baseline ZNRF3 mRNA or activity, or normal or baseline functional effect of RNF43 or ZNRF3 genes whether a patient sample, a cancer cell or a cell is more likely to be sensitive to or resistant to the Wnt inhibitor. The "control level" can refer to the sequence, parameter or level measured for comparison in a non-cancerous, healthy, wild-type tissue or cell, or in particular embodiment, placebo treated tumor cell.

The term "matched individuals" refers to a matching of the control individuals on the basis of one or more characteristics which are suitable for the type of cell or tumor growth to be evaluated. Control individuals can be matched with the patient to be evaluated on the basis of gender, age, race, or any relevant biological or sociological factor that may affect the baseline of the control individuals and the patient (*e.g.,* preexisting conditions, consumption of particular substances, levels of other biological or physiological factors).

A "pharmaceutical composition" is a combination of active agent and another carrier, *e.g.,* compound or composition, inert (for example, a detectable agent or label) or active, such as an adjuvant, diluent, binder, stabilizer, buffers, salts, lipophilic solvents, preservative, adjuvant or the like. Carriers also include pharmaceutical excipients and additives, for example; proteins, peptides, amino acids, lipids, and carbohydrates (*e.g.,* sugars, including monosaccharides and oligosaccharides; derivatized sugars such as alditols, aldonic acids, esterified sugars and the like; and polysaccharides or sugar polymers), which can be present singly or in combination, comprising alone or in combination 1-99.99% by weight or volume. Carbohydrate excipients include, for example; monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol. It can be solid or in a liquid form.

### Methods

In one aspect, the invention provides a method to select a cancer patient for treatment with a Wnt inhibitor, comprising testing a biological sample from the patient for the presence of an inactivating mutation in the RNF43 gene.

In one embodiment, the presence of the inactivating mutation is detected by DNA sequencing methods. In one embodiment, the presence of the inactivating mutation is detected by sequencing of genomic DNA, cDNA or RNA from a cancer tissue. In one embodiment, the presence of the inactivating mutation is detected by hybridization, polymerase chain reaction, polyacrylamide gel analysis, chromatography or spectroscopy. In one embodiment, the inactivating mutation is detected by screening for an altered protein product encoded by the gene, e.g. via immunoblot, Western blot, enzyme-linked immunosorbant assay (ELISA), radioimmunoassay (RIA), immunoprecipitation, immunohistochemistry, immunofluorescence, fluorescence activated cell sorting (FACS) and immunofluorescence microscopy.

According to the disclosure, the mutation status of RNF43 gene or ZNRF3 gene in the tumor cells can be assayed by any of the following methods, or by a combination of the methods.
(i) DNA copy number analysis of RNF43 region in chromosome 17 at genomic locus 17q22 to see whether one or both copies of the RNF43 gene are lost. Alternatively, DNA copy number analysis of the ZNRF3 region in chromosome 22 at genomic locus 22q12.1 to see whether one or both copies of the ZNRF3 gene are lost. The loss of heterozygosity of RNF43 or ZN RF3 is a biomarker for tumors that are selected for reduction in their rate of growth by treatment with a Wnt inhibitor.
(ii) sequencing of genomic DNA, cDNA or RNA from cancer tissues to detect an inactivation mutation in the RNF43 gene. An inactivation mutation of RNF43 or ZNRF3 is a biomarker for tumors that are selected for reduction in their rate of growth by treatment with a Wnt inhibitor. An inactivating mutation can be a nonsense mutation, frameshift mutation, a splicing variant or a missense mutation that results in amino acid change at conserved residues.
(iii) RNF43 mRNA expression assay or ZNRF3 mRNA expression assay using Taqman or other similar techniques. Nonsense or frameshift mutations in mRNAs often result in nonsense-mediated mRNA decay. Therefore, loss of RNF43 mRNA expression in cancer cells could be used as a secondary or alternative assay for RNF43 non-sense or frameshift mutations. Absence of RNF43 mRNA could also be due to epigenetic silencing, in which case there is no mutation at the genomic DNA.
(iv) Assaying the functional effect of RNF43 gene loss or ZNRF3 gene loss, such as by assaying for and detecting increased Frizzled protein levels, increased LRP6 protein levels, increased LRP6 phosphorylation, and increased Disheveled phosphorylation in tumor samples compared with normal control (wherein the increased Frizzled protein levels, increased LRP6 protein levels, increased LRP6 phosphorylation, and increased Disheveled phosphorylation in tumor samples is indicative of a lower than control level of the RNF43 gene biomarker or the ZNRF3 gene biomarker).

The step of detecting can be by using a nucleotide probe that hybridizes to the sequences provided in SEQ ID NO:1 (for RNF43) or SEQ ID NO:2 (for ZNRF3).

An inactivation mutation can be a nonsense mutation (see TABLE 1 and TABLE 2), a frameshift mutation (see TABLE 1 and TABLE 2), a splice site mutation or a missense mutation that results in amino acid change at conserved residues. FIG. 2 shows an alignment of the human RNF43 and ZNRF3 proteins, which provides a guide to which amino acids in the proteins are conserved.

A splice site mutation is a genetic mutation that inserts or deletes a number of nucleotides in the specific site at which splicing of an intron takes place during the processing of precursor messenger RNA into mature messenger RNA. The abolishment of the splicing site results in one or more introns remaining in mature mRNA and may lead to the production of aberrant proteins.

Nonsense or frameshift mutations in mRNAs often result in nonsense mediated mRNA decay. Therefore, loss of RNF43 mRNA expression in cancer cells could be used as a secondary or alternative assay for RNF43 non-sense or frameshift mutations. Absence of RNF43 mRNA could also be due to epigenetic silencing, in which case there is no mutation at the genomic DNA.

The step of comparing can be by comparing the biomarker level in the tumor cells to a control level of the biomarker in one or more control cells that are resistant to the Wnt inhibitor or in one or more control cells that are sensitive to the Wnt inhibitor, or both. In one aspect, the control level of the biomarker that has been correlated with sensitivity or resistance to the Wnt inhibitor has been predetermined.

For the step of selecting the patient as being predicted to benefit or not benefit from Wnt inhibitor therapy, it is contemplated that for the majority of patients tested, most patients will have tumors that are resistant to treatment by Wnt inhibitors. It is expected that the dependence of tumors on Wnt signaling is not as frequent as dependence of tumors on growth factor signaling. Moreover, if Wnt dependent tumors have genetic mutations downstream in the Wnt pathway, then the tumors would not respond to most current Wnt inhibitors. Thus, there is a need in the art to select for treatment the set of cancer patients who with be good responders to Wnt inhibitor treatment. The ability to predict good responders is the main utility of determining the RNF43 mutation status or ZNRF3 mutation status in tumors from cancer patients.

Any of the embodiments of the method of the invention described above can be used with a patient having any type of cancer. In one embodiment, the patient has a ductal carcinoma, adenocarcinoma or melanoma (see TABLE 1). In another embodiment, the patient has a pancreatic cancer, a colon cancer, a cancer of the esophagus or a skin cancer (see TABLE 1, TABLE 2, TABLE 3, TABLE 4, and TABLE 5). In yet another method of the invention, the patient has a gastric tumor with a RNF43 mutation.

In any of the embodiments of the invention described above, responsiveness to any Wnt inhibitor can be evaluated. *In vitro,* responsiveness of Wnt inhibitor can be determined in standard cell proliferation, differentiation, and apoptosis assays. The effect of Wnt inhibitor on the status of Wnt signaling in tumor cells can be assessed by checking the level of β-catenin, the mRNA expression of β-catenin target gene AXIN2, and phosphorylation of LRP6. In the clinic, evaluation is on the basis of the amount of tumor shrinkage by imaging if it is solid tumor or biomarker for tumor status, by a PET scan for example.

Methods of measurements for tumor volume are known in the art. See, Therasse P. et al. (2000) J. Natl. Cancer Inst. 92(3): 205-216. The same method of assessment and the same technique should be used to characterize each identified and reported lesion at baseline and during follow-up. Imaging-based evaluation is preferred to evaluation by clinical examination when both methods have been used to assess the antitumor effect of a treatment.

*Clinical examination.* Clinically detected lesions will only be considered measurable when they are superficial (*e.g.,* skin nodules and palpable lymph nodes). For the case of skin lesions, documentation by color photography-including a ruler to estimate the size of the lesion-is recommended.

*Chest x-ray.* Lesions on chest x-ray are acceptable as measurable lesions when they are clearly defined and surrounded by aerated lung. However, CT is preferable. More details concerning the use of this method of assessment for objective tumor response evaluation are provided by Therasse P et al. (2000) J. Natl. Cancer Inst. 92 (3): 205-216.

*CT and MRI.* X-ray computed tomography (CT) and magnetic resonance imaging (MRI) are the best currently available and most reproducible methods for measuring target lesions selected for response assessment. Conventional CT and MRI should be performed with contiguous cuts of 10 mm or less in slice thickness. Spiral CT should be performed by use of a 5-mm contiguous reconstruction algorithm; this specification applies to the tumors of the chest, abdomen, and pelvis, while head and neck tumors and those of the extremities usually require specific protocols. See, Therasse P et al. (2000) J. Natl. Cancer Inst. 92 (3): 205-216.

*Ultrasound.* When the primary end point of the study is objective response evaluation, ultrasound should not be used to measure tumor lesions that are clinically not easily accessible. It may be used as a possible alternative to clinical measurements for superficial palpable lymph nodes, subcutaneous lesions, and thyroid nodules. Ultrasound might also be useful to confirm the complete disappearance of superficial lesions usually assessed by clinical examination. Justifications for not using ultrasound to measure tumor lesions for objective response evaluation are provided in Appendix I.

*Endoscopy and laparoscopy.* The utilization of these techniques for objective tumor evaluation has not yet been fully or widely validated. Their uses in this specific context require sophisticated equipment and a high level of expertise that may be available only in some centers. Therefore, utilization of such techniques for objective tumor response should be restricted to validation purposes in specialized centers. However, such techniques can be useful in confirming complete histopathologic response when biopsy specimens are obtained.

*Tumor markers.* Tumor markers that are used for correlating with measurements for tumor volume (*i.e.,* not the markers of the invention) alone cannot be used to assess response. However, if markers are initially above the upper normal limit, they must return to normal levels for a patient to be considered in complete clinical response when all tumor lesions have disappeared. Specific additional criteria for standardized usage of prostate-specific antigen and CA (cancer antigen) 125 response in support of clinical trials are being validated.

*Cytology and histology.* Cytologic and histologic techniques can be used to differentiate between partial response and complete response in rare cases (e.g., after treatment to differentiate between residual benign lesions and residual malignant lesions in tumor types such as germ cell tumors). Cytologic confirmation of the neoplastic nature of any effusion that appears or worsens during treatment is required when the measurable tumor has met criteria for response or stable disease. Under such circumstances, the cytologic examination of the fluid collected will permit differentiation between response or stable disease (an effusion may be a side effect of the treatment) and progressive disease (if the neoplastic origin of the fluid is confirmed). New techniques to better establish objective tumor response will be integrated into these criteria when they are fully validated to be used in the context of tumor response evaluation.

### Loss of heterozygosity

The method for establishing a control level of loss of heterozygosity is selected based on the sample type, the tissue or organ from which the sample is obtained, and the status of the patient to be evaluated. The method can be the same method that will be used to evaluate the sample in the patient. The control level can be established using the same cell type as the cell to be evaluated. The control level can be established from control samples that are from patients or cell lines known to be resistant or sensitive to Wnt inhibitors. In one instance, the control samples were obtained from a population of matched individuals.

To establish a control level, samples from a number of matched individuals are obtained and evaluated in the same manner as for the test samples. The number of matched individuals from whom control samples must be obtained to establish a suitable control level (*e.g.,* a population) can be determined by those of skill in the art, but should be statistically appropriate to establish a suitable baseline for comparison with the patient to be evaluated (*i.e.,* the test patient). The values obtained from the control samples are statistically processed using any suitable method of statistical analysis to establish a suitable baseline level using methods standard in the art for establishing such values.

The copy number of the RNF43 gene or ZNRF3 gene per tumor cell can be detected by fluorescent *in situ* hybridization (FISH). FISH is a DNA-based technique and can be successfully performed in fresh or preserved paraffin-embedded tumor samples. The technology is well established and probes can readily be constructed with a short turnaround in clinical cytogenetics and molecular pathology laboratories. A FISH probe for mouse RNF43 is commercially available, so one of skill in the art would readily be able to construct a corresponding FISH probe for human RNF43. See, Rogan P et al.(2001) Genome Res. 11(6): 1086-1094. Alternatively, the service of custom design of a FISH probe for a human gene, such as human RNF43 or human ZNRF3, is commercially available, such as by FISH Probes from Empire Genomics, Buffalo, NY, USA.

### Hybridization

Detection of a gene can be accomplished using hybridization assays. Nucleic acid hybridization simply involves contacting a probe (*e.g.,* an oligonucleotide or larger polynucleotide) and target nucleic acid under conditions where the probe and its complementary target can form stable hybrid duplexes through complementary base pairing. As used herein, hybridization conditions refer to standard hybridization conditions under which nucleic acid molecules are used to identify similar nucleic acid molecules. Such standard conditions are disclosed, for example, in Sambrook et al. (1989) Molecular Cloning: A Laboratory Manual. Cold Spring Harbor Labs Press (incorporated by reference in its entirety, see specifically, pages 9.31-9.62). In addition, formulae to calculate the appropriate hybridization and wash conditions to achieve hybridization permitting varying degrees of mismatch of nucleotides are disclosed, for example, in Meinkoth et al. (1984) Anal. Biochem. 138, 267-284. Nucleic acids that do not form hybrid duplexes are washed away from the hybridized nucleic acids and the hybridized nucleic acids can then be detected, typically through detection of an attached detectable label. Nucleic acids are denatured by increasing the temperature or decreasing the salt concentration of the buffer containing the nucleic acids. Under low stringency conditions (*e.g.,* low temperature or high salt or both) hybrid duplexes (*e.g.,* DNA:DNA, RNA:RNA, or RNA:DNA) will form even where the annealed sequences are not perfectly complementary. Thus specificity of hybridization is reduced at lower stringency. Conversely, at higher stringency (*e.g.,* higher temperature or lower salt) successful hybridization requires fewer mismatches.

High stringency hybridization and washing conditions, as referred to herein, refer to conditions which permit isolation of nucleic acid molecules having at least about 90% nucleic acid sequence identity with the nucleic acid molecule being used to probe in the hybridization reaction (*i.e.,* conditions permitting about 10% or less mismatch of nucleotides). One of skill in the art can use the formulae in Meinkoth et al. (1984) Anal. Biochem. 138, 267-284 to calculate the appropriate hybridization and wash conditions to achieve these particular levels of nucleotide mismatch. Such conditions will vary, depending on whether DNA:RNA or DNA:DNA hybrids are being formed. Alternatively, Tₘ can be calculated empirically as set forth in Sambrook et al.(1989) Molecular Cloning: A Laboratory Manual. Cold Spring Harbor Labs Press., pages 9.31 to 9.62.

The hybridized nucleic acids are detected by detecting one or more labels attached to the sample nucleic acids. The labels may be incorporated by any of a number of means well known to those of skill in the art. Detectable labels suitable for use in the invention include any composition detectable by spectroscopic, photochemical, biochemical, immunochemical, electrical, optical or chemical means. Useful labels in the invention include fluorescent dyes (*e.g.,* fluorescein, Texas red, rhodamine, Alexa fluors, Spectrum dyes, and the like), quantum dots, radiolabels (*e.g.,* ³H, ¹²⁵I, ³⁵S, ¹⁴C, or ³²P), and colorimetric labels. Means of detecting such labels are well known to those of skill in the art. Thus, for example, radiolabels may be detected using photographic film or scintillation counters, fluorescent markers may be detected using a photodetector to detect emitted light and fluorescence microscopes. Colorimetric labels are detected by simply visualizing the colored label. Preferably, the hybridizing nucleic acids are detected by fluorescent labels and most preferably, in the context of a fluorescence *in situ* hybridization (FISH) assay. FISH assays are well known in the art.

### Inactivating mutation

It is disclosed herein that tumors having an inactivating mutation in the RNF43 gene orZNRF3 gene are more likely to be responsive to (*i.e.,* to having their growth slowed by) a Wnt inhibitor. Detection of one or more mutations in the RNF43 gene or ZNRF3 gene is predictive that the patient will benefit from treatment with the Wnt inhibitor. Guidance for this detection of one or more mutations in the RNF43 gene is provided in the EXAMPLES.

Detection of one or more mutations in the RNF43 gene is predictive that a patient is more likely to respond or benefit from Wnt inhibitor therapy. Detection of no mutations is predictive that a patient is less likely to respond or benefit from Wnt inhibitor therapy. Methods for screening for gene mutations are well-known in the art, are described in Sambrook et al. (1989) Molecular Cloning: A Laboratory Manual. Cold Spring Harbor Labs Press, and include hybridization, polymerase chain reaction, polyacrylamide gel analysis, chromatography or spectroscopy. With the recent advances being made in "next generation sequencing", it is contemplated that direct sequencing of polynucleotides is becoming the least expensive reliable method for assaying RNF43 gene mutation status or ZNRF3 gene mutation status.

Methods for screening for gene mutations can further include screening for an altered protein product encoded by the gene (*e.g.,* via immunoblot (*e.g.,* Western blot), enzyme-linked immunosorbant assay (ELISA), radioimmunoassay (RIA), immunoprecipitation, immunohistochemistry, immunofluorescence, fluorescence activated cell sorting (FACS) and immunofluorescence microscopy).

For loss of RNF43 mRNA, an at least a 50% drop in mRNA can be considered as indicating a loss of function, because tumor samples are not homogenously comprised of tumor cells.

### Patient sample

Suitable methods of obtaining a patient sample are known to a person of skill in the art. A patient sample can include any bodily fluid or tissue from a patient that may contain tumor cells or proteins of tumor cells.

In general, the sample type (*i.e.,* cell, tissue or bodily fluid) is selected based upon the accessibility and structure of the organ or tissue to be evaluated for tumor cell growth or upon what type of cancer is to be evaluated. The invention is particularly useful for evaluating a tumor such as a ductal carcinoma, adenocarcinoma or melanoma (see TABLE 1) or tumors from patients with pancreatic cancer, a colon cancer, a cancer of the esophagus or a skin cancer (see TABLE 1, TABLE 2, TABLE3, TABLE 4 and TABLE 5). In these cases, a typical sample is a section of a tumor sample or from a pancreatic tissue sample from the patient, respectively.

Once a sample is obtained from the patient, the sample is evaluated for detection of one or more of any of the biomarkers described herein. In some embodiments of the invention, a tissue, a cell or a portion thereof (*e.g.,* a section of tissue, a component of a cell such as nucleic acids, *etc*.) is contacted with one or more nucleic acids. Such protocols are used to detect gene expression or loss of heterozygosity, for example. Such methods can include cell-based assays or non-cell-based assays. The tissue or cell expressing a target gene is typically contacted with a detection agent (*e.g.,* a probe, primer, or other detectable marker), by any suitable method, such as by mixing, hybridizing, or combining in a manner that allows detection of the target gene by a suitable technique.

The patient sample is prepared by any suitable method for the detection technique utilized. In one embodiment, the patient sample can be used fresh, frozen, fixed or otherwise preserved. For example, the patient tumor cells can be prepared by immobilizing patient tissue in paraffin. The immobilized tissue can be sectioned and then contacted with a probe for detection of hybridization of the probe to a target gene (*e.g.,* RNF43 gene or the ZNRF3 gene).

### Controls

A control level need not be established for each assay as the assay is performed. Rather, a baseline or control can be established by referring to a form of stored information regarding a previously determined control level for sensitive and resistant patients (responders and non-responders). A control level can be established for any of the above-described detection methods, for use when a corresponding detection method is used. Such a form of stored information can include a reference chart, listing or electronic file of population or individual data regarding sensitive and resistant tumors/patients, or any other source of data regarding control level gene loss of heterozygosity that is useful for the patient to be evaluated.

A control level for comparison can be any type of control, including a preestablished control that is provided as a form of information. Other scoring systems can be devised based on comparisons with controls, and patients falling near the cut-off, can be evaluated by other criteria, biomarkers, or techniques in order to confirm a diagnosis. Also, the cut-off can be varied as desired by the clinician or investigator according to patient populations.

For a control that has been correlated with resistance to Wnt inhibitors, when the biomarker is an inactivating mutation in the RNF43 gene or the ZNRF3 gene, the control may be the wild-type sequence of the RNF43 gene or the ZNRF3 gene. Such sequences are publicly available and well defined. In one embodiment, the sequence of the RNF43 gene is provided by SEQ ID NO: 1. In another embodiment, the sequence of the ZNRF3 gene is provided by SEQ ID NO: 2.

For loss of RNF43 mRNA, at least a 50% decrease of mRNA expression in the tumor samples is to be observed.

Another control for resistance to Wnt inhibitors would be non-tumor samples from the same patient. If such non-tumor samples are not available, then averaged values from other patients or healthy subjects can be compared, because most patients would not respond to Wnt inhibitors.

### Wnt inhibitors

The method of the invention is useful for determining or predicting patients that are most likely to respond (*e.g.,* with a therapeutic benefit) to therapy using an Wnt inhibitor or a drug having substantially similar biological activity as the Wnt inhibitor, as well as to determine or predict patients that are most likely not to respond to therapy using an Wnt inhibitor. The disclosure also provides that cancer cells with RNF43 gene mutations are more sensitive to inhibition of the Wnt pathway. Inhibition of RNF43 in cancer cells leads to increased cell surface Frizzled levels. Accordingly, enhanced Wnt signaling and cancer cells, particularly pancreatic cancer cells, with mutant RNF43 are more sensitive to a Wnt antagonist. See, FIGS. 3 and 4, concerning the HPAFII, a cell line with a nonfunctional RNF43 protein. The inventors have also observed that the cell line Panc10.05, which also has a nonfunctional RNF43 protein, is also sensitive to Porcupine inhibitors.

In one embodiment, the Wnt inhibitor is a Porcupine inhibitor suitable for use in humans. The Wnt inhibitor may be a Porcupine inhibitor that has a function similar to a known Porcupine inhibitor such as IWP-2, IWP-3 or IWP-4, which are described by Chen B et al. (2009) Nature Chem. Biol. 5: 100-107 and commercially available from Miltenyi Biotech as Stemolecule^{™} Wnt Inhibitor IWP-2 (#130-095-584), Stemolecule^{™} Wnt Inhibitor IWP-3 (#130-095-585) and Stemolecule^{™} Wnt Inhibitor IWP-4. Stemolecule^{™} IWP-2, Stemolecule^{™} IWP-3, and Stemolecule^{™} IWP-4 prevent palmitylation of Wnt proteins by Porcupine (PORCN), a membrane-bound O-acyltransferase.

Alternatively, Wnt inhibitors can be the products of drug design and can be produced using various methods known in the art. See, international patent application WO2010/101849, published 10 September 2010. Various methods of drug design, useful to design mimetics or other compounds useful in the invention are disclosed in Maulik et al. (1997) Molecular Biotechnology: Therapeutic Applications and Strategies. Wiley-Liss, Inc.. A Wnt inhibitor can be obtained from molecular diversity strategies (a combination of related strategies allowing the rapid construction of large, chemically diverse molecule libraries), libraries of natural or synthetic compounds, in particular from chemical or combinatorial libraries (*i.e.,* libraries of compounds that differ in sequence or size but that have the similar building blocks) or by rational, directed or random drug design. See, for example, Maulik et al. (1997) Molecular Biotechnology: Therapeutic Applications and Strategies. Wiley-Liss, Inc.. In a molecular diversity strategy, large compound libraries are synthesized, for example, from peptides, oligonucleotides, natural or synthetic steroidal compounds, carbohydrates or natural or synthetic organic and non-steroidal molecules, using biological, enzymatic or chemical approaches. The critical parameters in developing a molecular diversity strategy include subunit diversity, molecular size, and library diversity. The general goal of screening such libraries is to utilize sequential application of combinatorial selection to obtain high-affinity ligands for a desired target, and then to optimize the lead molecules by either random or directed design strategies. Methods of molecular diversity are described in detail in Maulik et al.(1997) Molecular Biotechnology: Therapeutic Applications and Strategies. Wiley-Liss, Inc..

In a preferred embodiment the Wnt inhibitor is a compound of Formula (1): 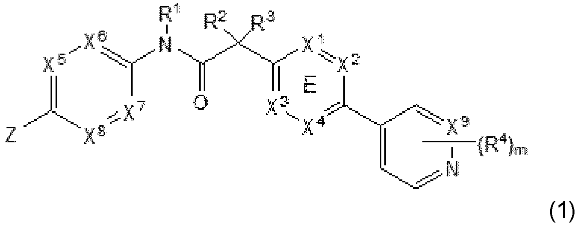 or a physiologically acceptable salt thereof, wherein:
X¹, X², X³ and X⁴ is selected from N and CR⁷;
one of X⁵, X⁶, X⁷ and X⁸ is N and the others are CH;
X⁹ is selected from N and CH;
Z is selected from phenyl, pyrazinyl, pyridinyl, pyridazinyl and piperazinyl; wherein each phenyl, pyrazinyl, pyridinyl, pyridazinyl or piperazinyl of Z is optionally substituted with an R⁶ group;
R¹, R² and R³ are hydrogen;
m is 1;
R⁴ is selected from hydrogen, halo, difluoromethyl, trifluoromethyl and methyl;
R⁶ is selected from hydrogen, halo and -C(O)R¹⁰; wherein R¹⁰ is methyl; and
R⁷ is selected from hydrogen, halo, cyano, methyl and trifluoromethyl.

The Wnt inhibitor can be a compound selected from the group of:
N-[5-(3-fluorophenyl)pyridin-2-yl]-2-[5-methyl-6-(pyridazin-4-yl)pyridin-3-yl]acetamide;
2-[5-methyl-6-(2-methylpyridin-4-yl)pyridin-3-yl]-N-[5-(pyrazin-2-yl)pyridin-2-yl]acetamide (LGK974);
N-(2,3'-bipyridin-6'-yl)-2-(2',3-dimethyl-2,4'-bipyridin-5-yl)acetamide;
N-(5-(4-acetylpiperazin-1-yl)pyridin-2-yl)-2-(2'-methyl-3-(trifluoromethyl)-2,4'-bipyridin-5-yl)acetamide;
N-(5-(4-acetylpiperazin-1-yl)pyridin-2-yl)-2-(2'-fluoro-3-methyl-2,4'-bipyridin-5-yl)acetamide; and
2-(2'-fluoro-3-methyl-2,4'-bipyridin-5-yl)-N-(5-(pyrazin-2-yl)pyridin-2-yl)acetamide;
or a pharmaceutically acceptable salt thereof.

Most preferably the Wnt inhibitor is 2-[5-methyl-6-(2-methylpyridin-4-yl)pyridin-3-yl]-N-[5-(pyrazin-2-yl)pyridin-2-yl]acetamide (LGK974).

A drug having substantially similar biological activity as a Wnt inhibitor refers to a drug having substantially the same functions exhibited or performed by the reference compound that is ascribed to the reference compound as measured or observed *in vivo* or *in vitro.* For example, a drug having substantially similar biological activity as a Porcupine inhibitor refers to a drug having substantially the same functions exhibited or performed by a reference compound such as IWP-2, IWP-3 or IWP-4.

In another embodiment, the Wnt inhibitor is the tankyrase inhibitor XAV939 (C9289), which is available from Sigma-Aldrich Corp., St. Louis, MO, USA, and other commercial sources. See, Huang SM et al. (2009) Nature 461(7264):614-20.

Other types of Wnt inhibitors can include, but are not limited to, aptamers, RNAi, and ribozymes. Aptamers are short strands of synthetic nucleic acids (usually RNA but also DNA) selected from randomized combinatorial nucleic acid libraries by virtue of their ability to bind to a predetermined specific target molecule with high affinity and specificity. Aptamers assume a defined three-dimensional structure and are capable of discriminating between compounds with very small differences in structure. RNA interference (RNAi) is a process whereby double stranded RNA, and in mammalian systems, short interfering RNA (siRNA), is used to inhibit or silence expression of complementary genes. A ribozyme is an RNA segment that is able to perform biological catalysis (e.g., by breaking or forming covalent bonds). More specifically, ribozymes are antisense RNA molecules that function by binding to the target RNA moiety and inactivate it by cleaving the phosphodiester backbone at a specific cutting site.

The other types of inhibitors may have similarity to the natural Wnt inhibitors. Known natural antagonists of Wnt signaling include Dickkopf proteins, secreted Frizzled-related proteins (sFRP), Wnt Inhibitory Factor 1 (WIF-1), and Soggy. Members of the Dickkopf-related protein family (Dkk-1 to -4) are secreted proteins with two cysteine-rich domains, separated by a linker region. The Dkk family also includes Soggy, which is homologous to Dkk-3 but not to the other family members.

The sFRPs are a family of five Wnt-binding glycoproteins that resemble the membrane-bound Frizzleds. The largest family of Wnt inhibitors, they contain two groups, the first consisting of sFRP-1, 2, and 5, and the second including sFRP-3 and 4.

In one embodiment, the antagonist of Wnt signaling can be a soluble Wnt receptor, such as a Frizzled8CRD-hFc, which is reported to have inhibited the growth of teratocarcinomas *in vivo.* DeAlmeida VI et al. (2007) Cancer Res. 67(11):5371-9.

Other natural antagonists of Wnt signaling include WIF-1 (Wnt Inhibitory Factor 1), a secreted protein that binds to Wnt proteins and inhibits their activity.

Yet another type of Wnt inhibitor can be an antibody, antigen binding fragment thereof, or an antigen binding peptide or "binding partner". Antibodies are characterized in that they comprise immunoglobulin domains and as such, they are members of the immunoglobulin superfamily of proteins. An antibody can include polyclonal and monoclonal antibodies, divalent and monovalent antibodies, bi- or multi-specific antibodies, serum containing such antibodies, antibodies that have been purified to varying degrees, and any functional equivalents of whole antibodies. Isolated antibodies useful as Wnt inhibitors can include serum containing such antibodies, or antibodies that have been purified to varying degrees. Whole antibodies of the invention can be polyclonal or monoclonal. Alternatively, functional equivalents of whole antibodies, such as antigen binding fragments in which one or more antibody domains are truncated or absent (*e.g.,* Fv, Fab, Fab', or F(ab)₂ fragments), as well as genetically-engineered antibodies or antigen binding fragments thereof, including single chain antibodies or antibodies that can bind to more than one epitope (*e.g.,* bi-specific antibodies), or antibodies that can bind to one or more different antigens (*e.g.,* bi- or multi-specific antibodies), may also be employed as Wnt inhibitors.

Various antibodies targeting upstream Wnt signaling are being developed, including LRP6 antibody (Ettenberg S et al. (2010) Proc. Natl. Acad. Sci. USA 107(35):15473-8) and Frizzled antibody (Gurney A et al. (2012) Proc. Natl. Acad. Sci. USA 109(29): 11717-22).

### Assays and kits

The assay kits and methods of the disclosure may be used to identify patient, cell, or tissue that is predicted to be responsive to a particular Wnt inhibitor. The use of such a companion diagnostic kit would be similar to other companion diagnostic tests approved by governmental drug registration agencies for use with approved drugs. See, for example, the approvals by the Food and Drug Administration in 2011 of crizotinib for the treatment of ALK4-mutated lung cancer and of vemurafenib for BRAF-mutated melanoma.

The assay kits and methods of the disclosure may also be useful for identifying treatments that can improve the responsiveness of cancer cells which are resistant to Wnt inhibitors, and to develop adjuvant treatments that enhance the response of the Wnt inhibitors.

The assay kits and methods of the disclosure are useful to patients with any cancer that can be treated with Wnt inhibitors, such as or pancreatic cancer (see TABLE 2), or any tumors whose growth can be slowed by Wnt inhibitors, such as ductal carcinomas, adenocarcinomas or melanomas (see TABLE 1). Such patients may, as a result of the methods provided herein, be spared from side effects and financial costs of an ineffective therapy in the event that they do not have a reduced gene expression of RNF43 or ZNRF3. The assay kits and methods of the disclosure are also useful to physicians, who can recommend, a Wnt inhibitor therapy, or not, to particular patients based on information on the molecular characteristics of their tumors. The assay kits and methods of the disclosure will also usefully increase the demand for development of an efficient human RNF43 FISH assay to be made available with yet-to-be developed nucleotide probes.

The kit can include a means for detecting a mutation in the RNF43 gene or ZNRF3 gene.

In one instance, the means for detecting the mutation is a nucleotide probe that hybridizes to a portion of the RNF43 gene or ZNRF3 gene. In one instance, the means for detecting is a fluorescent *in situ* hybridization (FISH) probe. Any of the means for detecting can contain a detectable label. Any of the means for detecting can be immobilized on a substrate.

In one instance, a means for detecting RNF43 gene or ZNRF3 gene loss of heterozygosity can generally be any type of reagent that can be used in a method of the disclosure. Such a means for detecting include a probe or primer that hybridizes under stringent hybridization conditions to RNF43 gene or ZNRF3. Nucleic acid sequences for the RNF43 gene or ZNRF3 are known in the art and can be used to produce such reagents for detection. Additional reagents useful for performing an assay using such means for detection can also be included, such as reagents for performing *in situ* hybridization, reagents for detecting fluorescent markers, reagents for performing polymerase chain reaction, *etc.*

The means for detecting of the assay kit of the disclosure can be conjugated to a detectable tag or detectable label. Such a tag can be any suitable tag which allows for detection of the reagents used to detect the gene or protein of interest and includes, but is not limited to, any composition or label detectable by spectroscopic, photochemical, electrical, optical or chemical means. Useful labels in the disclosure include: biotin for staining with labeled streptavidin conjugate, magnetic beads (*e.g.*, Dynabeads^{™}), fluorescent dyes (*e.g.,* fluorescein, texas red, rhodamine, green fluorescent protein, and the like), radiolabels (*e.g.,* 3H, ¹²⁵I, ³⁵S, ¹⁴C, or ³²P), enzymes (*e.g.,* horse radish peroxidase, alkaline phosphatase and others commonly used in an ELISA), and colorimetric labels such as colloidal gold or colored glass or plastic (*e.g.,* polystyrene, polypropylene, latex, *etc*.) beads.

In addition, the means for detecting of the assay kit of the disclosure can be immobilized on a substrate. Such a substrate can include any suitable substrate for immobilization of a detection reagent such as would be used in any of the previously described methods of detection. Briefly, a substrate suitable for immobilization of a means for detecting includes any solid support, such as any solid organic, biopolymer or inorganic support that can form a bond with the means for detecting without significantly affecting the activity or ability of the detection means to detect the desired target molecule. Exemplary organic solid supports include polymers such as polystyrene, nylon, phenolformaldehyde resins, and acrylic copolymers (*e.g.,* polyacrylamide). The kit may also include suitable reagents for the detection of the reagent or for the labeling of positive or negative controls, wash solutions, dilution buffers and the like. The kit may also include a set of written instructions for using the kit and interpreting the results.

The assay kit may also include one or more controls. The controls could include: (i) a control sample for detecting sensitivity to the Wnt inhibitor being evaluated for use in a patient; (ii) a control sample for detecting resistance to the Wnt inhibitor; (iii) information containing a predetermined control level of particular biomarker to be measured with regard to Wnt inhibitor sensitivity or resistance (*e.g.,* a predetermined control level of RNF43 gene or ZNRF3 gene loss of heterozygosity that has been correlated with sensitivity to the Wnt inhibitor or resistance to Wnt inhibitor).

The kit can also include a means for detecting a control marker that is characteristic of the cell type being sampled can generally be any type of reagent that can be used in a method of detecting the presence of a known marker (at the nucleic acid or protein level) in a sample, such as by a method for detecting the presence of a biomarker described previously herein. Specifically, the means is characterized in that it identifies a specific marker of the cell type being analyzed that positively identifies the cell type. For example, in a lung tumor assay, it is desirable to screen lung epithelial cells for the level of the biomarker expression or biological activity. Therefore, the means for detecting a control marker identifies a marker that is characteristic of an epithelial cell and preferably, a lung epithelial cell, so that the cell is distinguished from other cell types, such as a connective tissue or inflammatory cell. Such a means increases the accuracy and specificity of the assay of the disclosure. Such a means for detecting a control marker include, but are not limited to: a probe that hybridizes under stringent hybridization conditions to a nucleic acid molecule encoding a protein marker; PCR primers which amplify such a nucleic acid molecule; an aptamer that specifically binds to a conformationally distinct site on the target molecule; or an antibody, antigen binding fragment thereof, or antigen binding peptide that selectively binds to the control marker in the sample. Nucleic acid and amino acid sequences for many cell markers are known in the art and can be used to produce such reagents for detection.

### EXAMPLES

### Example 1

As shown in TABLE 1, the RNF43 gene is mutated in primary pancreatic ductal adenocarcinoma and other tumors. Nonsense and frameshift mutations in nine genomic DNA from primary tumors of pancreas, large intestine, esophagus and skin. Of them, five are pancreatic tumors and the total number of pancreatic tumors examined is 19 (mutated in over 25% of samples, excluding potentially damaging missense mutations).

| TABLE 1 | | | | | | |
|---|---|---|---|---|---|---|
| MUTATIO N | AA CHANG E | ID | PATHOLOGY | PRIMARY SITE | HISTOLOGY (SUBTYPE) | AG E |
| nonsense | p. R337X | X-1633 | primary | pancreas | carcinoma (ductal carcinoma) | 44 |
| frameshift | unknown | X-1948 | primary | pancreas | carcinoma (ductal carcinoma) | NA |
| frameshift | unknown | X-2406 | primary | pancreas | carcinoma (ductal carcinoma) | NA |
| frameshift | unknown | X-3184 | primary | pancreas | carcinoma (ductal carcinoma) | 55 |
| frameshift | unknown | X-3268 | primary | pancreas | carcinoma (ductal carcinoma) | 78 |
| frameshift | unknown | X-2239 | primary | large intestine | carcinoma (ductal carcinoma) | 76 |
| frameshift | unknown | X-3205 | primary | large intestine | carcinoma (adeno-carcinoma) | 78 |
| frameshift | unknown | X-1433 | metastasi s | esophagus | carcinoma (adeno-carcinoma) | 78 |
| nonsense | p.W302X | X-2163 | metastasi s | skin | melanoma (NS) | 54 |

### Example 2

As shown in TABLE 2, the RNF43 gene is mutated in multiple pancreatic cancer cell lines. Genomic variations identified by Sanger sequencing in 10 pancreatic cancer cell lines potentially with only one copy of RNF43 gene left based on copy number analysis. Three unique cell lines with RNF43 inactivating mutations were identified: HPAFII (nonsense mutation), Panc 10.05 (frameshift mutation, related with PL45 cells), PaTu-8988S (detrimental mutation, related with PaTu-8988T cells).

| TABLE 2 | | | | | | |
|---|---|---|---|---|---|---|
| CELL LINE | GENE NAME | cDNA CHANGE | EXON | AA CHANGE | Conservation | Comments |
| PK-1 | RNF43 | *c.g350a* | 2 | p.R117H | No | |
| HPAFII | RNF43 | *c.g520t* | 4 | p.E174X | Yes | Non-functional |
| Panc 10.05 | RNF43 | *c*.54*in*satca | 1 | p.M18fs | ~ | Non-functional |
| PL45 | RNF43 | *c*.54*in*satca | 1 | p.M18fs | ~ | Non-functional |
| PaTu-8988S | RNF43 | *c.t206g* | 1 | p.F69C | Yes | Non-functional |
| PaTu-8988T | RNF43 | *c.t206g* | 1 | p.F69C | Yes | Non-functional |
| KLM-1 | | *c.g350a* | 2 | p.R117H | | |
| Capan-1 | RNF43 ! | *c.c692t* | 6 | p.P231L | No | Both non-polar and hydrophobic |
| KP1N | RNF43 | *c.c692t* | 6 | p.P231L | | Both non-polar and hydrophobic |
| PANC-1 | RNF43 | *c.c692t* | 6 | p.P231L | No | Both non-polar and hydrophobic |

Additional information about the HPAFII, Panc 10.05, PaTu-8988S cell lines, as well as other cell lines with RNF43 inactivating mutations (*e.g.,* Capan-2) are provided in Example 3 and Example 4 below.

### Example 3

### Canonical Wnt pathway inhibition by LGK974

The sensitivity of pancreatic cells to LGK974 treatment were tested in a cellular proliferation assay *in vitro.* Twenty four human pancreatic cancer cell lines were treated with or without LGK974.

Cellular proliferation data was generated in 384 well format. Cells were harvested and resuspended in their appropriate growth medium at a density of 1.5 X 10⁴ cells per mL. Cells were then plated into 384 well tissue culture plates (Greiner-BioOne 789163) at a final volume of 50µL per well to achieve a per well density of 750 cells per well using a BioTek µFill dispenser (Serial number 000-3586). Plates were then transferred to incubators on the ACP-1 system (37°C, 5% CO₂) and cells were allowed to attach overnight. A 12 point dose response curve for LGK974 was prepared in a 384 well ECHO compatible source plate (Labcyte P-05525) with a highest concentration of 2 mM and a lowest concentration of 1.13 × 10⁻⁵ mM. Approximately 18 hours after plating the cells were dosed with 50 nL of LGK974 using the Labcyte ECHO555, with replicate IC₅₀ curves within a plate and replicate plates per cell line. Following compound addition, plates were returned to the incubator for 120 hours. The assay plates were read with the addition of 10 µL of 1x Cell Titer Glo (Promega G7573) according to the manufacturer's instructions. Plates were then incubated at room temperature for ten minutes and read on the integrated Perkin Elmer Viewlux (2 second exposure, 2x bin, high sensitivity). The raw data was normalized using the DMSO control wells within each plate and the curve fitting for IC₅₀ determination was performed.

Cell growth was measured five days later with Cell Titer Glo (Promega). As shown in TABLE 3, LGK974 inhibited cell growth with nM IC₅₀'s in a subset of pancreatic cancer cell lines, including Capan-2, PA-TU-8988S and HPAFII. As shown in TABLE 4 and Example 4, all four cell lines harbor RNF43 loss-of-function (LOF) mutations

| TABLE 3 | | |
|---|---|---|
| Cell Line | LGK974 IC50s in cell line proliferation assay | Inhibition fold change (1-Treated/Control) |
| Capan-2 | 0.0018 | 0.40 |
| PA-TU-8988S | 0.0116 | 0.40 |
| HPAFII | 0.0336 | 0.50 |
| TCC-PAN2 | >1.7 | 0.02 |
| BXPC3 | >2.0 | 0.01 |
| HUP-T3 | >2.0 | 0.07 |
| KP-1N | >2.0 | 0.01 |
| KP-1NL | >2.0 | 0.00 |
| KP-2 | >2.0 | 0.06 |
| KP-3 | >2.0 | 0.02 |
| Panc 03.27 | >2.0 | 0.06 |
| Panc 05.04 | >2.0 | 0.09 |
| Panc 10.05 | >2.0 | 0.02 |
| Panel | >2.0 | 0.00 |
| PA-TU-8902 | >2.0 | 0.02 |
| PA-TU-8988T | >2.0 | 0.00 |
| PK-1 | >2.0 | 0.00 |
| PK-59 | >2.0 | 0.06 |
| PSN-1 | >2.0 | 0.06 |
| SU8686 | >2.0 | 0.03 |
| SUIT-2 | >2.0 | 0.00 |
| SW1990 | >2.0 | 0.03 |
| YAPC | >2.0 | 0.20 |

TABLE 4 shows a list of pancreatic cell lines, the RNF43 mutations in the cell lines and the pathway inhibition by LGK974

| TABLE 4 | | | |
|---|---|---|---|
| Sample ID (Cell line) | Potential LOF mutation | Proliferation IC₅₀ₛ | Pathway inhibition |
| Capan-2 | p.R330fs | 0.0018 | Yes |
| HPAFII | p. E174X | 0.0336 | Yes |
| PA-Tu-8988S | F69C | 0.0116 | Yes |
| PA-TU-8988T | F69C | >2.0 | Yes |
| Panc 10.05 | p. M18fs | >2.0 | Yes |
| PL45 | p. M18fs | NA | No |
| BXPC3 | p. S495Y | >2.0 | Yes |
| KP1N | None | >2.0 | Yes |
| PK-1 | None | >2.0 | Yes |
| PANC-1 | None | >2.0 | Yes |
| Panc03.27 | None | >2.0 | Yes |
| SUIT-2 | None | >2.0 | Yes |
| YAPC | None | >2.0 | Yes |
| PANC-05-04 | None | >2.0 | Yes |
| KP-2 | None | >2.0 | Yes |
| KP-3 | None | >2.0 | Yes |
| PA-TU-8902 | None | >2.0 | Yes |
| PK-59 | None | >2.0 | Yes |
| SW1990 | None | >2.0 | Yes |
| Tcc-Pan2 | None | >2.0 | Yes |
| HUP-T3 | None | >2.0 | Yes |
| PA-TU-8902 | None | >2.0 | Yes |
| SU8686 | None | >2.0 | Yes |
| KP-1NL | None | >2.0 | Yes |
| SW1990 | None | >2.0 | Yes |
| PK-59 | None | >2.0 | Yes |
| PSN-1 | None | >2.0 | Yes |
| HUP-T3 | None | >2.0 | Yes |
| Capan-1 | None | NA | No |
| KLM-1 | None | NA | NA |
| HuCCT1 | None | NA | NA |
| MiaPaca | None | NA NA | No |
| PK-45H | None | NA | No |
| DANG | None | NA | No |
| Panc-04-03 | None | NA | No |
| QGP-1 | None | NA | No |

### Example 4

### Inactivation Mutation of RNF43 Confers Wnt-dependency in Pancreatic Cancer

In this Example, we show that RNF43 inhibits Wnt/β-catenin signaling and reduces the membrane level of Frizzled in pancreatic cancer cells as a negative feedback mechanism. Inhibition of endogenous Wnt signaling increased the cell surface level of Frizzled.

Multiple pancreatic cancer cell lines were tested for Wnt dependency using LGK974, a Porcupine inhibitor that blocks Wnt secretion. Pancreatic cancer cells were grown in medium recommended by ATCC supplemented with 10% FBS.

Strikingly, all Porcupine inhibitor sensitive lines carry inactivating mutation of RNF43. Inhibition of Wnt secretion or depletion of β-catenin inhibits proliferation of RNF43 mutant but not RNF43 WT pancreatic tumor cells. Reintroduction of wild type RNF43 into RNF43 mutant lines also inhibits their proliferation. LGK974 inhibits the growth of RNF43 mutant pancreatic tumors *in vivo.* Our data show that mutation of RNF43 in pancreatic cancer confers Wnt-dependency and RNF43 mutation should be used a biomarker for patient selection for the development of Wnt inhibitors.

Three Wnt-dependent pancreatic cancer cell lines have been identified using Porcupine inhibitor LGK974, and strikingly all these cell lines harbor RNF43 loss-of-function mutations. The growth of these RNF43 mutant cell lines is inhibited upon β-catenin depletion or RNF43 re-expression, and their growth is inhibited by LGK974. Our data establish RNF43 as a tumor suppressor in pancreatic cancer, and show that RNF43 mutation can be used as a biomarker for predicting efficacy of agents targeting Wnt signaling pathway.

We tested a panel of 39 pancreatic cancer cell lines for Wnt-dependency using LGK974, a Porcupine inhibitor currently being examined in clinics. Strikingly, all the LGK974-sensitive lines carry inactivating mutations of RNF43. Inhibition of Wnt secretion, depletion of β-catenin, or expression of wild-type RNF43 blocked proliferation of RNF43 mutant but not RNF43 wild-type pancreatic cancer cells. LGK974 inhibited the growth of RNF43 mutated pancreatic tumors in mouse xenograft models.

| TABLE 5 | | |
|---|---|---|
| | Growth inhibition in foci formation assay | Pathway inhibition in Axin2 qPCR assay |
| BxPC3 | No | Yes |
| Capan-1 | No | No |
| Capan-2* | Yes | Yes |
| CFPAC-1 | No | No |
| DanG | No | No |
| HPAFII* | Yes | Yes |
| Hs766T | No | ND |
| HuPT3 | No | Yes |
| HuPT4 | No | No |
| KCI-MOH1 | No | ND |
| KLM1 | No | ND |
| KP1-N | No | Yes |
| KP-1NL | No | Yes |
| KP2 | No | Yes |
| KP3 | No | Yes |
| KP4 | No | No |
| L3.3 | No | No |
| MIA CaPa-2 | No | No |
| PANC-1 | No | Yes |
| Pan02.03 | No | ND |
| Panc03.27 | No | Yes |
| Panc04.03 | No | No |
| Panc05.04 | No | Yes |
| Panc08.13 | No | ND |
| Panc10.05* | No | Yes |
| PaTu-8902 | No | Yes |
| PaTu-8988S* | Yes | Yes |
| PaTu-8988T* | No | Yes |
| PK45H | No | No |
| PK1 | No | Yes |
| PK59 | No | Yes |
| PL45* | No | No |
| PSN-1 | No | Yes |
| QGP-1 | No | No |
| SU86.86 | No | Yes |
| SUIT-2 | No | Yes |
| SW1990 | No | Yes |
| T3M4 | No | No |
| YAPC | No | Yes |
| * cell lines with inactivating mutation of RNF43 | | |
| ND: Not Determined | | |

To assay foci formation, 6,000-12,000 cells of indicated cell lines were seeded in 6 well tissue culture plate in 2ml growth media. After overnight culture for cell attachment, media was replaced with fresh growth media containing 1µM LGK974 in the absence and presence of recombinant Wnt3a. For DOX-inducible β-catenin shRNA experiment, cells were treated 5ng/ml doxycycline. When cell colonies reached desirable size, cells were fixed with 4% formalin in PBS and stained with crystal violet solution. After a few washes, plates were dried and imaged.

For reverse transcription and quantitative PCR (qPCR), total RNA was extracted from cells or tumors using the RNeasy Plus Mini Kit (Qiagen). 1µg of RNA were reverse transcribed with Taqman Reverse Transcription Reagents (Applied Biosystems) according to the manufacturer's instruction. Quantitative PCR was performed in 12µl reactions consisting of 0.6µl of 20X Taqman probe and PCR primer mix, 6µl 2X Taqman FAST Advanced Master Mix (Applied Biosystems), and 5.4µl diluted cDNA template. The thermocycling conditions utilized were 20 seconds at 95°C, followed by 40 cycles of 1second at 95°C and 20 seconds at 60°C. All experiments were performed in quadruplicates. Gene expression analysis was performed using the comparative ΔΔCT method and normalized with the housekeeping gene GUSB or 18S. The Taqman probes were purchased from Applied Biosystems.

### Negative regulation of Wnt signaling by RNF43 in pancreatic cancer cells.

*Depletion of RNF43 increases Wnt-induced STF.* Since RNF43 is frequently mutated in cystic pancreatic tumors (Furukawa T et al.(2011) Sci. Rep. 1:161; Wu J et al. (2011) Proc. Natl. Acad. Sci. USA 108(52):21188-93), RNF43 could be an essential regulator of Wnt/β-catenin signaling in pancreatic cancer cells. Accordingly, we performed loss of function experiments in YAPC, a pancreatic cancer cell line. Depletion of RNF43 using independent siRNAs significantly increased SuperTOPFlash (STF) Wnt reporter activity, either in the absence or in the presence of exogenous Wnt3a conditioned medium. See, FIG. 3(a).

For the luciferase reporter assay, YAPC-STF reporter cells were transfected with siRNA and treated with Wnt3a conditioned medium or compound where applicable. STF luciferase assays were performed using BrightGlo Luciferase Assay kits (Promega) according to the manufacturer's instructions.

To construct pancreatic cell lines expressing STF reporter, HEK293 cells were grown in medium recommended by ATCC supplemented with 10% FBS. Retrovirus or lentivirus was produced from HEK293 cells by standard virus packaging procedure using FuGENE 6 (Roche) transfection reagent. Pancreatic cell lines expressing STF reporter, RNF43 constructs, or DOX-inducible β-catenin shRNA were generated by viral infection and drug selection.

siRNA transfection was performed using Dharmafect 1 transfection reagent (Dharmacon) according to manufacturer's instruction. Sequences of siRNAs are listed as follows: pGL2 (Dharmacon D-001100-01), target sequence, 5' - CGTACGCGGAATACTTCGA -3'; RNF43-1 (Dharmacon J-007004-12), target sequence, 5' -GGUGGAGUCUGAAAGAUCA-3'; RNF43-2 (Dharmacon J-007004-11), target sequence, 5' -GGAGAAAGCUAUUGCACAG -3'; CTNNB1- 1 (Dharmacon J-003482-10), target sequence, 5' -UAAUGAGGACCUAUACUUA -3'; and CTNNB1- 2 (Dharmacon J-003482-12), target sequence, 5' -GGUACGAGCUGCUAUGUUC-3'.

*Depletion of RNF43 induces Dvl phosphorylation and β-catenin stabilization.* LGK974 is a Porcupine inhibitor that potently inhibits Wnt secretion and is currently under clinical evaluation. RNF43 siRNA-induced STF activity in the absence of exogenous Wnt3a is inhibited by LGK974 and a previously known Porcupine inhibitor IWP-2. Chen B et al. (2009) Nat. Chem. Biol. 5(2): 100-7. See, FIG. 3(b), showing that this activity is dependent on the expression of endogenous Wnt proteins. These results show that RNF43 actively suppresses autocrine Wnt/β-catenin signaling in YAPC cells.

*Depletion of RNF43 increases FZD level by FACS.* We next performed biochemical assays to characterize the function of RNF43 in YAPC cells. Depletion of RNF43 increased the level of cytosolic β-catenin, in agreement with increased STF reporter activity.

Dishevelled (DVL) is an intracellular signaling protein downstream of Frizzled, and its phosphorylation is stimulated by Frizzled. Depletion of RNF43 increased phosphorylation of DVL2 in an immunohistochemistry assay. Anti-DVL antibody was obtained from Cell Signaling Technology, Danvers, MA, USA.

Indeed, depletion of RNF43 drastically increased the cell surface level of Frizzled, as assayed by flow cytometry using pan-Frizzled antibody 18R5. TABLE 6 shows that depletion of RNF43 increases the cell surface level of Frizzled (FZD). YAPC cells were transfected with indicated siRNA, and membrane levels of Frizzled were analyzed by flowcytometry using pan-Frizzled antibody 18R5.

| TABLE 6 | |
|---|---|
| Negative regulation of Wnt signaling by RNF43 in pancreatic cancer cells | |
| siRNA | Median FZD intensity in FACS |
| pGL2 siRNA | 237 |
| RNF43 siRNA#1 | 1498 |
| RNF43 siRNA#2 | 1751 |
| Unstained | 33 |

For flow cytometric analysis, cells were harvested using trypsin-free cell dissociation buffer (Invitrogen) and resuspended in FACS buffer (PBS with 1% BSA and 0.02% sodium azide). After blocking, cells were incubated with anti-pan-Frizzled (18R5) antibody for 1 hour at 4 °C, followed by incubation with Allophycocyanin (APC)-conjugated goat anti-human IgG secondary antibody. After extensive washes using FACS buffer, cells were stained with propidium iodide (PI) and subjected to multi-channel analysis using BD LSR II flow cytometer. Fluorescence signals from PI-negative cells were displayed in histogram plots.

*Overexpression of wild type RNF43 decreases membrane FZD.* To rule out the possibility that the effect of RNF43 siRNA is mediated by off-target activity, we performed cDNA rescue experiment by stably expressing siRNA- resistant RNF43 cDNA in YAPC cells.

Expression of siRNA-resistant RNF43 largely abolished the effect of RNF43 siRNA on Frizzled levels, so the activity of RNF43 siRNA is on-target.

*Overexpression of RNF43ΔRING increases membrane FZD.* Depletion of RNF43 also increased the expression of AXIN2, a β-catenin target gene, and this effect is also abolished by the expression of siRNA resistant RNF43. See, FIG. 3(c).

RNF43 cDNA-resistant to RNF43 siRNA, RNF43 ΔRING (missing amino acids 272-312) and F69C mutant were generated by two-step mutagenesis PCR and cloned into various mammalian expression vectors. pLenti6-STF reporter plasmid and β-catenin shRNA viral plasmid were previously described by Hao H-X et al. (2012) Nature 485(7397):195-200.

On the basis of the threshold cycle (Ct) values that we observed in a quantitative PCR assay, RNF43 is dominantly expressed compared to ZNRF3 in YAPC cells. Our results show that RNF43 negatively regulates Wnt signaling through decreasing membrane expression of Frizzled in pancreatic cells.

*RNF43 is expressed at higher level than ZNRF3 in pancreatic cancer YAPC by qPCR, consistent with its importance in regulating Wnt signaling.* Wnt/β-catenin signaling promotes the development of the exocrine compartment of pancreas and ectopic expression of stabilized β-catenin proliferation of acinar cells. Heiser PW et al. (2006) Development 133(10):2023-32. We have previously shown that R-spondin proteins stimulate Wnt signaling through inhibiting ZNRF3 and RNF43 and stabilizing Frizzled. Hao HX et al. (2012) Nature 485(7397): 195-200.

### Wnt/β-catenin signaling suppresses the membrane expression of Frizzled

*Wnt*/ *β-catenin signaling negatively regulates FZD membrane expression. Porcupine inhibitor LGK974 increases the membrane level of FZD.* RNF43 and ZNRF3 have been shown to be β-catenin target genes. Hao HX et al. (2012) Nature 485(7397):195-200; Koo BK et al. (2012) Nature 488(7413):665-9. However, the effect of endogenous Wnt/β-catenin signaling on Frizzled expression has not been tested.

We found by flow cytometry that independent β-catenin siRNAs significantly increased the cell surface level of Frizzled in YAPC cells.. Depletion of β-catenin also decreased the mRNA levels of β-catenin targeting gene AXIN2 and RNF43. See, FIG. 4(a). TABLE 7 shows that depletion of β-catenin increases the cell surface level of Frizzled. YAPC cells were transfected by indicated siRNA, and membrane levels of Frizzled were analyzed by flowcytometry.

| TABLE 7 | |
|---|---|
| Wnt/β-catenin signaling suppresses membrane expression of Frizzled in pancreatic cancer cells | |
| siRNA | Median FZD intensity in FACS |
| pGL2 siRNA | 137 |
| P-catenin siRNA#1 ! | 530 |
| β-catenin siRNA#2 | 561 |
| Unstained | 37 |

Consistent with this observation, treatment of Porcupine inhibitor IWP2 or LGK974 increased the cell surface level of Frizzled and decreased the mRNA levels of AXIN2 and RNF43. See, FIG. 4(b). TABLE 8 shows that Porcupine inhibitors increases the cell surface level of Frizzled. YAPC cells were treated with 3 µM of IWP-2 or 1 µM of LGK974, and subjected to flowcytometry analysis for membrane Frizzled expression.

| TABLE 8 | |
|---|---|
| Wnt/β-catenin signaling suppresses membrane expression of Frizzled in pancreatic cancer cells | |
| Compound | Median FZD intensity in FACS |
| DMSO | 721 |
| IWP-2 | 1754 |
| LGK974 | 1373 |
| Unstained | 64 |

These results show that Wnt/β-catenin signaling strongly inhibits the membrane level of Frizzled.

### Characterization of RNF43 mutation in pancreatic tumors

*Identification of pancreatic cancer lines containing inactivation mutation of RNF43.* Discovery of LGK974, a potent and selective inhibitor of Porcupine, offered us a unique chemical tool to systemically examine Wnt dependency in a large panel of cancer cell lines. We tested LGK974 in a large panel of pancreatic cancer cell lines using foci formation assay. Foci formation assay was utilized because it is more sensitive than routine growth assays such as CellTiter-Glo, and it is less affected by the different growth rates of various cell lines. Of the 39 pancreatic cell lines screened, LGK974 only showed strong growth inhibitory activity in three cell lines, PaTu-8988S, HPAFII and Capan-2.

We next sought to determine the genetic lesion that could confer Wnt dependency. RNF43 is the only known negative regulator in the upstream of Wnt pathway mutated in cancer. For this reason, we performed RNF43 exon sequencing in all pancreatic cancer cell lines. Strikingly, all three LGK974-sensitive cell lines have homozygous mutations of RNF43 (HPAFII, E174X; PaTu-8988S, F69C; Capan-2, R330fs). E174X and R330fs mutations truncate the majority of RNF43 protein and most likely inactivate the protein. The consequence of F69C mutation, which introduces an extra cystine to the extracellular domain of RNF43, is less clear.

The mutations in the cell lines were determined by genomic sequencing analysis. For sequencing analysis generally, genomic DNA was extracted using QIAamp DNA Mini Kit (Qiagen) according to the manufacturer's instructions. Exons of RNF43 were amplified by PCR and sequenced using the Applied Biosystems platform.

To define the function of F69C mutation, we stably expressed C-terminal HA-tagged wild-type RNF43, RNF43 ΔRING, and RNF43 F69C in YAPC cells.

Full length human RNF43 cDNA (NM_017763.4) was purchased from Open Biosystems and tagged with a C-terminal HA epitope by PCR. RNF43 cDNA-resistant to RNF43 siRNA, RNF43 ΔRING (missing amino acids 272-312) and F69C mutant were generated by two-step mutagenesis PCR and cloned into various mammalian expression vectors. pLenti6-STF reporter plasmid and β-catenin shRNA viral plasmid were previously described by Hao H-X et al. (2012) Nature 485(7397):195-200.

Consistent with the function of RNF43 in the regulation of Frizzled turnover, overexpression of wild-type RNF43 decreased the membrane level of Frizzled, while overexpression of RNF43 ΔRING showed dominant negative activity and increased the membrane level of Frizzled. Overexpression of RNF43 F69C modestly increased the membrane level of Frizzled, so F69C is a loss of function mutant and has partial dominant negative activity upon overexpression.

TABLE 9 shows the flow cytometric analysis of membrane Frizzled in YAPC cells stably expressing empty vector (EV), wild-type (WT) RNF43, or mutant (ΔRING or F69C) RNF43.

| TABLE 9 | |
|---|---|
| Cells | RNF43 re-expression |
| EV | 1040 |
| Wild-type | 686 |
| ΔRING | 2004 |
| F69C | 1501 |
| Unstained | 64 |

Further, overexpression of RNF43 ΔRING, and to a less degree for RNF43 F69C, increased DVL2 phosphorylation and potentiates Wnt3a-induced STF reporter activity. These data demonstrate that the F69C is an inactivating missense mutation for RNF43.

*Depletion of RNF43 increases FZD membrane expression in RNF43 wild-type, but not RNF43 mutant cell.* We next examined the function of RNF43 in these pancreatic cancer cell lines. Although depletion of RNF43 increased DVL2 phosphorylation in YAPC and PK1, two pancreatic cancer cell lines with wild-type RNF43, depletion of RNF43 in HPAFII, PaTu-8988S, and Capan-2 cells did not increase DVL2 phosphorylation. Consistently, depletion of RNF43 increased the cell surface level of Frizzled in RNF43 wild-type (YAPC and PK1), but not in RNF43 mutant (HPAFII, PaTu-8988S, and Capan-2) pancreatic cancer cell lines. Taken together, these results show that Frizzled level is no longer inhibited by RNF43 in pancreatic cancer cell lines with RNF43 mutation.

TABLE 10 shows a flow cytometric analysis of membrane Frizzled in pancreatic cancer cell lines treated with RNF43 siRNA.

| TABLE 10 | | | |
|---|---|---|---|
| Mean FZD intensity in FACS | | | |
| Cells | pGL2 siRNA | RNF43 siRNA | unstained |
| | 353 | 1224 | 32 |
| PK1 | 473 | 923 | 32 |
| HPAFII | 375 | 402 | 23 |
| PaTu-8988S | 293 | 245 | 34 |
| Capan-2 | 2437 | 2643 | 23 |

### RNF43 mutation predicts sensitivity to Wnt inhibition in pancreatic tumors.

*LGK974 decreases cytosolic β-catenin in all cell lines.* We next characterized Wnt-dependency in pancreatic cancer cell lines. In foci formation assay, LGK974 strongly inhibited the growth of PaTu-8988S, HPAFII, and Capan-2, without significant effect on PK1 and YAPC (see TABLE 6). Importantly, the growth inhibitory effect of LGK974 in RNF43 mutant cells is rescued by exogenous Wnt3a, so the effect of LGK974 is mediated by blocking of Wnt secretion. (Pancreatic cancer cell lines were treated with DMSO, 1 µM of LGK974, or LGK974 together with recombinant Wnt3a in foci formation assay.) Immunoblot and qPCR assays indicated that LGK974 decreased the expression of MYC, a β-catenin target gene, and increased the expression of cyclin dependent kinase inhibitor p21 in RNF43 mutant, but not RNF43 wild-type cell lines. LGK974 decreased cytosolic β-catenin and decreased the expression of β-catenin target gene AXIN2 in both RNF43 mutant and RNF43 wild-type cell lines, so all these cell lines have active autocrine Wnt signaling.

Further, LGK974 induced the expression of differentiation marker MUC2, MUC5A/C in RNF43 mutant cells. LGK974 blocked EDU incorporation in RNF43 mutant cell lines, so Wnt inhibition in these cells leads to cell cycle arrest. LGK974-induced growth inhibition and cellular differentiation of RNF43 mutant cells are also obvious using Ki67 staining (anti-Ki67 (SP6) from Vector Laboratories, Burlingame, CA, USA 94010) and Alcian Blue staining. Further, LGK974 strongly inhibited the proliferation of PaTu-8988S and HPAFII , but not PK1, in soft agar assay. Capan-2 cannot be tested as it does not grow in soft agar assay. Together, these results show that inhibition of autocrine Wnt signaling in RNF43 mutant cells induces growth arrest and cellular differentiation.

For soft agar assay, cells were suspended in 250µl of 0.3% low melting agarose (Lonza) in DMEM containing 10% FBS, and plated onto 250µl of solidified 0.8% agarose containing DMEM in 48-well culture plates at a density of 5,000-10,000 cells per well. 250µl of growth medium were added on top of the cells after plate was cooled at Rt for 30 minutes. The plate was then incubated at 37 °C in a humidified atmosphere containing 5% CO₂. The cells were treated with fresh growth medium containing DMSO or 1 µM LGK974 every 3-4 days. When colonies reached desirable size, photographs were taken and colonies were stained with AlamarBlue (from Invitrogen, Life Technologies, Grand Island, NY, USA 14072) according to the manufacturer's instruction. These experiments were repeated three times, and at least four wells were replicated each time for each condition.

For the EdU proliferation assay, cells were plated in growth medium in 96 well plate at a density of 6000-12,000 cells per well, and treated with DMSO or 1µM LGK974. After 3 days, the cells were treated with fresh growth medium containing 20µM EdU, which was included in the Click-iT EdU Alexa Fluor 488 HCS assay kit (Invitrogen), and the plate was incubated for 2 hr at 37 °C in a humidified atmosphere containing 5% CO₂. Cells were fixed with final 4% paraformaldehyde for 30 min, washed with PBS, permeabilized, and stained with 0.75% TritonX-100 and 50µg/ml Hoechst in PBS for 30 min. After wash, the cells were proceeded EdU detection according to the instruction of Click-iT EdU assay kit. Triplet wells were performed for each condition.

The procedure for the Alcian blue staining for mucin is as follows: Pancreatic cancer cell lines were plated onto 225 cm³ tissue culture flasks at various densities, and were treated with DMSO or LGK974 (100 nM) for 72 hours. Cell pellets were harvested by removing media, washing with 1x PBS, and adding 10 ml of 10% buffer formalin. Cells were then scraped from the bottom of the flask and placed in a 50 ml conical tube, filled to ~50 ml with 10% buffered formalin, and allowed to fix for 1-2 hours. The conical tube containing the fixed cells was then centrifuged at 1200 rpm for 5 min, and the pelleted cells were wrapped in lens paper, placed in a histology cassette, processed, and paraffin embedded. FFPE sections were cut at 5 µm, mounted on slides, baked at 60oC for at least 30 minutes, and deparaffinized. Slides were then rinsed two times in H₂O, transferred to Acetic Acid 3% Aqueous for 3 minutes, and moved directly to Alcian Blue 1% in 3% acetic acid pH 1 for 30 minutes. Slides were then placed in running water for 10 minutes after which they were rinsed in distilled H₂O before being placed in Nuclear Fast Red 0.1% (Kernechtrot) for 5 minutes. Slides were again washed in running water, and then dehydrated. Lastly, the slides were coverslipped with Permaslip^{®}.

For immunoblotting, total cell lysates were prepared by lysing cells using RIPA buffer (50 mM Tris-HCI, pH 7.4, 150 mM NaCl, 1% NP-40, 0.5% sodium deoxycholate, 0.1% SDS, 1 mM EDTA) supplemented with protease inhibitors and phosphatase inhibitors, followed by centrifugation at 14,000 rpm for 10 min at 4°C. For cytosolic β-catenin extraction, cell pellets were resuspended in hypotonic buffer (10mM Tris-HCI, pH 7.5 and 10mM KCI, supplemented with protease/phosphatase inhibitors) and lysed by three freeze-thaw cycles. Equal amount of proteins were resolved by SDS-PAGE, transferred to nitrocellulose membranes, and incubated with primary antibodies for overnight at 4°C. Secondary antibodies conjugated with either HRP or infrared dyes were used for signal visualization by ECL film or LI-COR Odyssey scanner, respectively.

*LGK974 inhibits MYC and induces p21 protein expression in RNF43 mutant but not RNF43 wild-type pancreatic cancer lines.* Frizzled proteins potentiate both canonical Wnt/β-catenin signaling and noncanonical Wnt signaling, so mutational inactivation of RNF43 would conceivably increase both canonical and noncanonical Wnt signaling. To determine the contribution of Wnt/β-catenin signaling in cell growth of the pancreatic lines we studied, we used β-catenin shRNA. Inducible expression of a previously validated β-catenin shRNA (Scholer-Dahirel A et al. (2011) Proc. Natl. Acad. Sci. USA 108(41): 17135-40) strongly inhibited the proliferation of RNF43 mutant (PaTu-8988S, HPAFII, and Capan-2), but not RNF43 wild-type (PK1 and YAPC) pancreatic cancer lines. Depletion of β-catenin also decreased the expression of β-catenin target gene AXIN2 and MYC (anti-MYC is available from Abcam, Cambridge, MA, USA 02139), and increased the expression of p21 (anti-p21 is available from Millipore, Bedford, MA, USA 01730), MUC2, and MUC5A/Cs in RNF43 mutant cell lines. These results show that the effect of LGK974 on cell growth is likely mediated by canonical Wnt/β-catenin signaling.

We next examined whether RNF43 mutation is required for the growth of RNF43 mutant cells. We found that expression of wild-type RNF43, but not LacZ or RNF43 ΔRING, significantly inhibited the proliferation of RNF43 mutated PaTu-8988S and Capan-2 cells, while had no effects on RNF43 wild-type YAPC cells. Overexpression of wild-type RNF43 presumably overcomes the minor dominant activity of endogenously expressed RNF43 F69C in PaTu-8988S cells. HPAFII cannot be tested in this assay since viral infection efficiency of this cell line is too low and we could not obtain stably infected cells using retrovirus expressing LacZ or RNF43 ΔRING.

Together, our results show that Wnt/β-catenin signaling enhanced by RNF43 mutation is required for the proliferation of RNF43 mutant pancreatic cancer cells, and that suppression of Wnt/β-catenin signaling in these cells induces cell cycle arrest and induction of differentiation markers.

*Blocking Wnt secretion inhibits the growth of RNF43 mutant pancreatic tumors in vivo.* To analyze the role of Wnt pathway activation in maintenance of RNF43 mutant pancreatic tumors *in vivo,* we utilized two PDAC xenograft models (HPAFII and Capan-2)

For *in vivo* efficacy and pharmacodynamic studies, cells were harvested at a density of 95-110% confluency. 10 million cells (HPAFII) or 3 million cells (Capan-2) mixed 50:50 with BD matrigel matrix basement membrane (Matrigel) (BD Biosciences) were subcutaneously implanted into the upper right supra-axillary region of nu/nu (Harlan) (HPAFII) or scid.bg (Harlan) (Capan-2) mice. Tumors were monitored twice weekly by calipering and tumor volumes (TV) were calculated using the ellipsoid formula: TV(mm³) = ((I x w²) × 3.14159)) / 6. Xenograft tumor-bearing mice were randomized to treatment groups (n=8 per group) either 14 days post tumor implant (Capan-2), when the mean tumor volume reached 223 mm³ (range 200-250 mm³) or 11 days post implant (HPAFII), when the mean tumor volume reached 341 mm³ (range 272-418 mm³). Mice were treated by oral gavage (p.o.) twice daily (BID) at a dosing volume of 10 mL/kg with either vehicle (0.5% methylcelluose (MC) / 0.5% Tween 80) or a 0.65 mg/mL suspension of the fumarate salt form of LGK974 (LGK974-AE-4, free base molecular weight conversion factor 1.293) in 0.5% MC/ 0.5% Tween 80. *In vivo* doses are reported as free base equivalents. Following treatment for 14 days (HPAFII) or 35 days (Capan-2), anti-tumor activity was reported as percent treatment/control (%T/C) or %Regression (%REG) values. Anti-tumor activity was calculated using the following formula: %T/C = 100 ΔΔTₜ/ΔCₜ if ΔTₜ ≥ 0; or %REG = 100 ΔΔTₜ/T₀ if ΔTₜ < 0; where: T₀ = mean tumor volume (TV) of the drug-treated group on the day of randomization; Tₜ = mean TV of the drug-treated group at study end; ΔTₜ = T₀ - Tₜ; Cₜ = mean TV of the control group on the final day of the study; C₀ = mean TV of the control group on the day of randomization; and ΔCt = Co - Ct * %T/C values in the range of 100 to 42% are interpreted to have no anti-tumor activity; %T/C values ≤ 42% and >10% are interpreted to have anti-tumor activity, %T/C values ≤ 10% or %REG ≥ - 10% are interpreted to be tumor stasis. %REG values < -10% are interpreted as regressions. Separate, parallel cohorts of animals (n=3 per treatment and time point) were treated with vehicle or LGK974 as above to obtain tumor tissue for *ex vivo* analysis of pharmacodynamic markers.

Treatment of mice bearing HPAFII xenografts with 5 mg/kg LGK974, p.o. BID for 14 days resulted in a significant inhibition of tumor growth (T/C=33%) relative to vehicle treatment. Furthermore, treatment of mice bearing Capan-2 xenografts with 5 mg/kg LGK974, p.o. BID for 35 days achieved tumor stasis (T/C=5%). Consistent with the mechanism of action of LGK974, expression of the β-catenin target gene, AXIN2, was decreased in treated HPAFII and Capan-2 xenografts. Similar to *in vitro* findings in RNF43 mutant PDAC cells, treatment with LGK974 induced cell cycle arrest and differentiation within the xenograft tumors.

For immunohistochemistry and image analyses, xenograft tumor samples were fixed in 10% neutral-buffered formalin for 6-24 hours, processed, and paraffin embedded. Immunohistochemical staining was performed on the Ventana Discovery System. Images were captured using Aperio Scanscope. Images of whole sections of mouse pancreata and xenograft tumors were analyzed with Visiopharm. For xenograft tumors, stromal tissue was automatically excluded using the Section Assembler module. Necrotic regions were manually excluded using the drawing tools provided by the analysis software. Tissues were segmented using the TissuemorphDP module, and DAB intensity was quantified as either percent positive nuclei for nuclear proteins or percent positive pixels for proteins not confined to the nucleus.

### Discussion.

In this Example, we have demonstrated that RNF43 serves as a negative feedback regulator of Wnt pathway in pancreatic cells by suppressing membrane expression of Frizzled. We have shown that Wnt/β-catenin signaling potently inhibits Frizzled membrane expression, likely through induction of RNF43. This finding provides an explanation why pancreatic cancer cells select to mutate RNF43 to escape from this powerful negative feedback regulation and achieve high level of Wnt/β-catenin signaling. Our data establish RNF43 as a tumor suppressor in pancreatic cancer, and show that RNF43 mutation can be used as a biomarker for predicting efficacy of agents targeting Wnt signaling pathway.

We have found that LGK974 decreases the expression of AXIN2 in 22 of 29 (76%) pancreatic cancer cell lines, so a high percentage of pancreatic cancer cell lines have autocrine Wnt signaling. However, the growth of most of these cell lines is not affected by LGK974 in foci formation assay. Strikingly, all three pancreatic cancer lines that show clear sensitivity to LGK974 in growth assay carry RNF43 loss of function mutation. These results show that RNF43 mutated tumors have much higher chance to be Wnt dependent as compared to RNF43 wild-type tumors.

Interestingly, not all cell lines with RNF43 mutations are sensitive to LGK974. We have found three pancreatic cancer lines carrying homozygous mutation of RNF43, Patu-8988T (F69C), Panc10.05 (M18fs), and PL45 (M18fs), but the growth of these cell lines is not sensitive to LGK974. Note that Patu-8988S and Patu-8988T were derived from the same patient, and Panc10.05 and PL45 were also derived from the same patient. These results show that other mechanisms can render RNF43 mutated tumors independent of Wnt signaling.

Enriching patients who are most likely to respond to a therapy in clinical trials is beneficial for the successful development of molecularly targeted cancer therapeutics, because of the toxicity of these agents and the heterogeneity of tumors at the molecular lesion level. However, the clinical development of these agents is difficult because a good strategy to identify Wnt- dependent tumors is not available. A strategy based on overexpression of Wnt proteins or underexpression of Wnt inhibitors would not be robust enough for patient selection. Indeed, many PDAC lines with clear autocrine Wnt/β-catenin signaling are not dependent on Wnt for *in vitro* growth. Furthermore, β-catenin signaling can be activated in a ligand independent manner, so a strategy based on nuclear accumulation of β-catenin would not be reliable either.

Our study has established RNF43 as a tumor suppressor that inhibits upstream Wnt signaling in pancreatic cancer. Our finding that all Wnt inhibitor pancreatic cancer cell lines sensitive to Wnt inhibitor *in vitro* carry RNF43 mutation show that RNF43 mutation can be used as a predicative biomarker for selecting pancreatic tumors for a clinical trial to test Wnt inhibitor efficacy.

## Claims

1. A method for selecting a cancer patient for treatment with a Wnt inhibitor, comprising testing bodily fluid or tissue from a patient that may contain tumor cells or proteins of tumor cells for the presence of an inactivating mutation in the RNF43 gene.

2. The method of claim 1, wherein the presence of the inactivating mutation is detected by DNA sequencing methods.

3. The method of claim 1 or 2, wherein the presence of the inactivating mutation is detected by sequencing of genomic DNA, cDNA or RNA from a cancer tissue.

4. The method of claim 1, wherein the presence of the inactivating mutation is detected by hybridization, polymerase chain reaction, polyacrylamide gel analysis, chromatography or spectroscopy.

5. The method of claim 1, wherein the inactivating mutation is detected by screening for an altered protein product encoded by the gene, e.g. via immunoblot, Western blot, enzyme-linked immunosorbant assay (ELISA), radioimmunoassay (RIA), immunoprecipitation, immunohistochemistry, immunofluorescence, fluorescence activated cell sorting (FACS) and immunofluorescence microscopy.

6. A Wnt inhibitor or a pharmaceutical composition comprising a Wnt inhibitor for use in the treatment of cancer in a patient, wherein Wnt inhibitor is administered to the patient on the basis that an inactivating mutation in the RNF43 gene is detected in a bodily fluid or tissue from a patient that may contain tumor cells or proteins of tumor cells, and wherein the presence of an inactivating mutation in the RNF43 gene is detected using the method of any one of claims 2-5.

7. The Wnt inhibitor or a pharmaceutical composition comprising a Wnt inhibitor for use according to claim 6, wherein the cancer is pancreatic cancer, ductal carcinoma, adenocarcinoma or melanoma.

8. The Wnt inhibitor or the pharmaceutical composition comprising a Wnt inhibitor for use according to claim 6, wherein the cancer is pancreatic cancer.

9. The method of any one of claims 1 to 5, or the Wnt inhibitor or the pharmaceutical composition for use according to claim 6, 7, or 8, wherein the Wnt inhibitor is a compound of Formula (1): 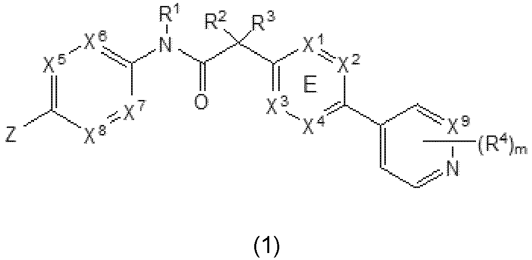 or a physiologically acceptable salt thereof, wherein:
X¹, X², X³ and X⁴ is selected from N and CR⁷;
one of X⁵, X⁶, X⁷ and X⁸ is N and the others are CH;
X⁹ is selected from N and CH;
Z is selected from phenyl, pyrazinyl, pyridinyl, pyridazinyl and piperazinyl;
wherein each phenyl, pyrazinyl, pyridinyl, pyridazinyl or piperazinyl of Z is optionally substituted with an R⁶ group;
R¹, R² and R³ are hydrogen;
m is 1;
R⁴ is selected from hydrogen, halo, difluoromethyl, trifluoromethyl and methyl;
R⁶ is selected from hydrogen, halo and -C(O)R¹⁰; wherein R¹⁰ is methyl; and
R⁷ is selected from hydrogen, halo, cyano, methyl and trifluoromethyl.

10. The method of any of of claims 1 to 5, or the Wnt inhibitor or the pharmaceutical composition for use according to any one of claims 6 to 9, wherein the Wnt inhibitor is a compound selected from the group of
N-[5-(3-fluorophenyl)pyridin-2-yl]-2-[5-methyl-6-(pyridazin-4-yl)pyridin-3-yl]acetamide;
2-[5-methyl-6-(2-methylpyridin-4-yl)pyridin-3-yl]-N-[5-(pyrazin-2-yl)pyridin-2-yl]acetamide;
N-(2,3'-bipyridin-6'-yl)-2-(2',3-dimethyl-2,4'-bipyridin-5-yl)acetamide ;
N-(5-(4-acetylpiperazin-1-yl)pyridin-2-yl)-2-(2'-methyl-3-(trifluoromethyl)-2,4'-bipyridin-5-yl)acetamide;
N-(5-(4-acetylpiperazin-1-yl)pyridin-2-yl)-2-(2'-fluoro-3-methyl-2,4'-bipyridin-5-yl)acetamide; and
2-(2'-fluoro-3-methyl-2,4'-bipyridin-5-yl)-N-(5-(pyrazin-2-yl)pyridin-2-yl)acetamide ;
or a pharmaceutically acceptable salt thereof.

11. The method of any one of claims 1 to 5, or the Wnt inhibitor or the pharmaceutical composition for use according to any one of claims 6 to 9, wherein the Wnt inhibitor is 2-[5-methyl-6-(2-methylpyridin-4-yl)pyridin-3-yl]-N-[5-(pyrazin-2-yl)pyridin-2-yl]acetamide.

## Patentansprüche

1. Verfahren zur Auswahl eines Krebspatienten für eine Behandlung mit einem Wnt-Inhibitor, umfassend Testen von Körperflüssigkeit oder Gewebe von einem Patienten, die bzw. das möglicherweise Tumorzellen oder Proteine von Tumorzellen enthält, auf das Vorliegen einer inaktivierenden Mutation im RNF43-Gen.

2. Verfahren nach Anspruch 1, wobei das Vorliegen der inaktivierenden Mutation mit DNA-Sequenziermethoden nachgewiesen wird.

3. Verfahren nach Anspruch 1 oder 2, wobei das Vorliegen der inaktivierenden Mutation durch Sequenzieren von genomischer DNA, cDNA oder RNA aus einem Krebsgewebe nachgewiesen wird.

4. Verfahren nach Anspruch 1, wobei das Vorliegen der inaktivierenden Mutation durch Hybridisierung, Polymerase-Kettenreaktion, Polyacrylamidgel-Analyse, Chromatographie oder Spektroskopie nachgewiesen wird.

5. Verfahren nach Anspruch 1, wobei die inaktivierende Mutation durch Screening auf ein durch das Gen codiertes verändertes Proteinprodukt, z. B. über Immunoblot, Western-Blot, ELISA (Enzyme-linked Immunosorbant Assay), RIA (Radioimmunoassay), Immunpräzipitation, Immunhistochemie, Immunfluoreszenz, FACS (Fluorescence Activated Cell Sorting) und Immunfluoreszenzmikroskopie nachgewiesen wird.

6. Wnt-Inhibitor oder einen Wnt-Inhibitor umfassende pharmazeutische Zusammensetzung zur Verwendung bei der Behandlung von Krebs bei einem Patienten, wobei dem Patienten Wnt-Inhibitor auf der Basis verabreicht wird, dass eine inaktivierende Mutation im RNF43-Gen in einer Körperflüssigkeit bzw. einem Gewebe von einem Patienten, die bzw. das möglicherweise Tumorzellen oder Proteine von Tumorzellen enthält, nachgewiesen wird, und wobei das Vorliegen einer inaktivierenden Mutation im RNF43-Gen unter Verwendung des Verfahrens nach einem der Ansprüche 2-5 nachgewiesen wird.

7. Wnt-Inhibitor oder einen Wnt-Inhibitor umfassende pharmazeutische Zusammensetzung zur Verwendung gemäß Anspruch 6, wobei es sich bei dem Krebs um Bauchspeicheldrüsenkrebs, duktales Karzinom, Adenokarzinom oder Melanom handelt.

8. Wnt-Inhibitor oder einen Wnt-Inhibitor umfassende pharmazeutische Zusammensetzung zur Verwendung gemäß Anspruch 6, wobei es sich bei dem Krebs um Bauchspeicheldrüsenkrebs handelt.

9. Verfahren nach einem der Ansprüche 1 bis 5 oder Wnt-Inhibitor oder pharmazeutische Zusammensetzung zur Verwendung gemäß Anspruch 6, 7 oder 8, wobei es sich bei dem Wnt-Inhibitor um eine Verbindung der Formel (1): 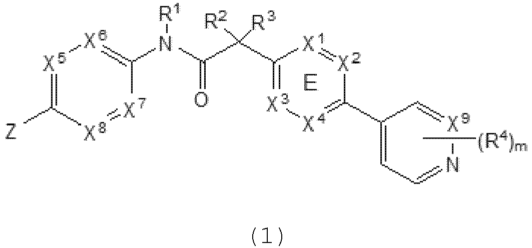 oder ein physiologisch unbedenkliches Salz davon handelt, wobei:
X¹, X², X³ und X⁴ jeweils aus N und CR⁷ ausgewählt ist;
einer von X⁵, X⁶, X⁷ und X⁸ für N steht und die anderen für CH stehen;
X⁹ aus N und CH ausgewählt ist;
Z aus Phenyl, Pyrazinyl, Pyridinyl, Pyridazinyl und Piperazinyl ausgewählt ist; wobei jedes Phenyl, Pyrazinyl, Pyridinyl, Pyridazinyl oder Piperazinyl von Z gegebenenfalls mit einer R⁶-Gruppe substituiert ist;
R¹, R² und R³ jeweils für Wasserstoff stehen;
m gleich 1 ist;
R⁴ aus Wasserstoff, Halogen, Difluormethyl, Trifluormethyl und Methyl ausgewählt ist;
R⁶ aus Wasserstoff, Halogen und -C(O)R¹⁰, wobei R¹⁰ für Methyl steht, ausgewählt ist; und
R⁷ aus Wasserstoff, Halogen, Cyano, Methyl und Trifluormethyl ausgewählt ist.

10. Verfahren nach einem der Ansprüche 1 bis 5 oder Wnt-Inhibitor oder pharmazeutische Zusammensetzung zur Verwendung gemäß einem der Ansprüche 6 bis 9, wobei es sich bei dem Wnt-Inhibitor um eine Verbindung ausgewählt aus der Gruppe
N-[5-(3-Fluorphenyl)pyridin-2-yl]-2-[5-methyl-6-(pyridazin-4-yl)pyridin-3-yl]acetamid;
2-[5-Methyl-6-(2-methylpyridin-4-yl)pyridin-3-yl]-
N-[5-(pyrazin-2-yl)pyridin-2-yl]acetamid;
N-(2,3'-Bipyridin-6'-yl)-2-(2',3-dimethyl-2,4'-bipyridin-5-yl)acetamid;
N-(5-(4-Acetylpiperazin-1-yl)pyridin-2-yl)-2-(2'-methyl-3-(trifluormethyl)-2,4'-bipyridin-5-yl)-acetamid;
N-(5-(4-Acetylpiperazin-1-yl)pyridin-2-yl)-2-(2'-fluor-3-methyl-2,4'-bipyridin-5-yl)acetamid; und
2-(2'-Fluor-3-methyl-2,4'-bipyridin-5-yl)-N-(5-(pyrazin-2-yl)pyridin-2-yl)acetamid;
oder ein pharmazeutisch unbedenkliches Salz davon handelt.

11. Verfahren nach einem der Ansprüche 1 bis 5 oder Wnt-Inhibitor oder pharmazeutische Zusammensetzung zur Verwendung gemäß einem der Ansprüche 6 bis 9, wobei es sich bei dem Wnt-Inhibitor um 2-[5-Methyl-6-(2-methyl-pyridin-4-yl)pyridin-3-yl]-N-[5-(pyrazin-2-yl)pyridin-2-yl]acetamid handelt.

## Revendications

1. Procédé pour la sélection d'un patient cancéreux pour un traitement par un inhibiteur de Wnt, comprenant un test d'un fluide ou tissu corporel provenant d'un patient qui peut contenir des cellules tumorales ou des protéines de cellules tumorales en ce qui concerne la présence d'une mutation inactivante dans le gène RNF43.

2. Procédé selon la revendication 1, la présence de la mutation inactivante étant détectée par des procédés de séquençage d'ADN.

3. Procédé selon la revendication 1 ou 2, la présence de la mutation inactivante étant détectée par séquençage d'ADN, d'ADNc ou d'ARN génomique provenant d'un tissu cancéreux.

4. Procédé selon la revendication 1, la présence de la mutation inactivante étant détectée par hybridation, réaction en chaîne par polymérase, analyse sur gel de polyacrylamide, chromatographie ou spectroscopie.

5. Procédé selon la revendication 1, la présence de la mutation inactivante étant détectée par criblage pour un produit de protéine modifiée codé par le gène, par ex. via un immunotransfert, un transfert western, un dosage immunoenzymatique lié (ELISA), un dosage radioimmunologique (RIA), une immunoprécipitation, une immunohistochimie, une immunofluorescence, un tri de cellules activées par fluorescence (FACS) et une microscopie d'immunofluorescence.

6. Inhibiteur de Wnt ou composition pharmaceutique comprenant un inhibiteur de Wnt pour une utilisation dans le traitement d'un cancer chez un patient, l'inhibiteur de Wnt étant administré au patient sur la base du fait qu'une mutation inactivante dans le gène RNF43 est détectée dans un fluide ou tissu corporel d'un patient qui peut contenir des cellules tumorales ou des protéines de cellules tumorales, et la présence d'une mutation inactivante dans le gène RNF43 étant détectée en utilisant le procédé selon l'une quelconque des revendications 2 à 5.

7. Inhibiteur de Wnt ou composition pharmaceutique comprenant un inhibiteur de Wnt pour une utilisation selon la revendication 6, le cancer étant un cancer pancréatique, un carcinome ductal, un adénocarcinome ou un mélanome.

8. Inhibiteur de Wnt ou composition pharmaceutique comprenant un inhibiteur de Wnt pour une utilisation selon la revendication 6, le cancer étant un cancer pancréatique.

9. Procédé selon l'une quelconque des revendications 1 à 5, ou inhibiteur de Wnt ou composition pharmaceutique pour une utilisation selon la revendication 6, 7 ou 8, l'inhibiteur de Wnt étant un composé de formule (1) : 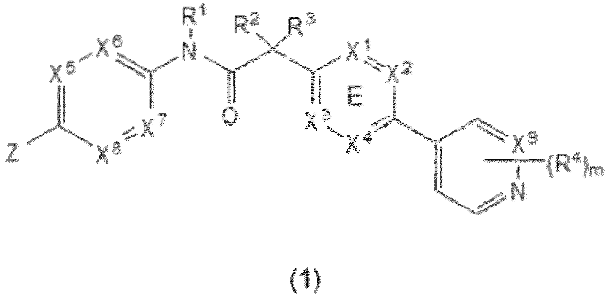 ou un sel physiologiquement acceptable correspondant,
X¹, X², X³ et X⁴ étant choisis parmi N et CR⁷ ;
l'un parmi X⁵, X⁶, X⁷ et X⁸ étant N et les autres étant CH ;
X⁹ étant choisi parmi N et CH ;
Z étant choisi parmi phényle, pyrazinyle, pyridinyle, pyridazinyle et pipérazinyle ; chaque phényle, pyrazinyle, pyridinyle, pyridazinyle ou pipérazinyle de Z étant éventuellement substitué par un groupe R⁶ ;
R¹, R² et R³ étant hydrogène ;
m étant 1 ;
R⁴ étant choisi parmi hydrogène, halogène, difluorométhyle, trifluorométhyle et méthyle ;
R⁶ étant choisi parmi hydrogène, halogéno et - C(O)R¹⁰ ; R¹⁰ étant méthyle ; et
R⁷ étant choisi parmi hydrogène, halogéno, cyano, méthyle et trifluorométhyle.

10. Procédé selon l'une quelconque des revendications 1 à 5, ou inhibiteur de Wnt ou composition pharmaceutique pour une utilisation selon l'une quelconque des revendications 6 à 9, l'inhibiteur de Wnt étant un composé choisi dans le groupe composé de
N-[5-(3-fluorophényl)pyridin-2-yl]-2-[5-méthyl-6-(pyridazin-4-yl)pyridin-3-yl]acétamide ;
2-[5-méthyl-6-(2-méthylpyridin-4-yl)pyridin-3-yl]-
N-[5-(pyrazin-2-yl)pyridin-2-yl]acétamide ;
N-(2,3'-bipyridin-6'-yl)-2-(2',3-diméthyl-2,4'-bipyridin-5-yl)acétamide ;
N-(5-(4-acétylpipérazin-1-yl)pyridin-2-yl)-2-(2'-méthyl-3-(trifluorométhyl)-2,4'-bipyridin-5-yl)acétamide ;
N-(5-(4-acétylpipérazin-1-yl)pyridin-2-yl)-2-(2'-fluoro-3-méthyl-2,4'-bipyridin-5-yl)acétamide ; et
2-(2'-fluoro-3-méthyl-2,4'-bipyridin-5-yl)-N-(5-(pyrazin-2-yl)pyridin-2-yl)acétamide ;
ou un sel pharmaceutiquement acceptable correspondant.

11. Procédé selon l'une quelconque des revendications 1 à 5, ou inhibiteur de Wnt ou composition pharmaceutique pour une utilisation selon la revendication 6 à 9, l'inhibiteur de Wnt étant le 2-[5-méthyl-6-(2-méthylpyridin-4-yl)pyridin-3-yl]-N-[5-(pyrazin-2-yl)pyridin-2-yl]acétamide.
